# EUROPEAN PATENT APPLICATION

(11) **EP 4 362 028 A1**
(43) Date of publication of application: **01.05.2024**
(21) Application number: 22204815.9
(22) Date of filing: 31.10.2022
(51) Int. Cl.: G16B 30/00, C07K 14/195

(54) **MUTANT AEROLYSIN AND USES THEREOF**

(71) Applicant: ECOLE POLYTECHNIQUE FEDERALE DE LAUSANNE (EPFL), 1015 Lausanne (CH)
(72) Inventor: CAO, Chan, 1015 Lausanne (CH); DAL PERARO, Matteo, 1015 Lausanne (CH)
(74) Representative: KATZAROV S.A.

(57) **Abstract**

Provided herein are aerolysin polypeptides and/or mutant aerolysin monomers comprising modified amino acid sequences that could have improved substrate analyte, such as (poly)nucleotide and peptide, improved reading properties such as enhanced substrate analyte capture and improved substrate analyte recognition and/or discrimination. Also provided herein are aerolysin pores derived from the mutant monomers. Also provided herein are apparatuses and devices comprising modified aerolysin polypeptides. Also provided herein are methods of using modified aerolysin proteins and pores derived therefrom for use in characterizing and/or sequencing a polymeric molecule or for use as molecular sensors.

## Description

### Technical Field

The invention relates to mutant forms of aerolysin. The invention also relates to analyte, polynucleotide or polypeptide transport and characterisation using the mutant forms of aerolysin.

### Background Art

Using biological nanopores to sequence biopolymers, particularly nucleic acids, was proposed many years ago. Recent advances in enzyme-based control of DNA translocation and in DNA nucleotide resolution using mutated biological pores have satisfied the needs for a functional DNA sequencing biological device.

Nanopore sensing is an approach that relies on the exploitation of individual binding or interaction events between to-be-analysed molecules and pore-forming macromolecules. Nanopore sensors can be created by placing nanometric-scaled pore peptide structures in an insulating membrane and measuring voltage-driven ionic transport through the pore in the presence of substrate molecules. The identity of a substrate can be ascertained through its peculiar electric signature, particularly the duration and extent of current block and the variance of current levels. Two of the essential components of sequencing nucleic acids using nanopore sensing are (1) the control of nucleic acid movement through the pore and (2) the discrimination of nucleotides as the nucleic acid polymer is moved through the pore.

Pore-forming proteins are produced by a variety of organisms and are often involved in defence or attack mechanisms. One notable feature is that they are produced as soluble proteins that subsequently oligomerize and convert into a transmembrane pore in the target membrane. The most extensively characterized pore-forming proteins are the bacterial pore-forming toxins (PFTs), which, depending on the secondary structure elements that cross the bilayer, have been classified as *α* - or *β* -PFTs.

In the past, to achieve nucleotide discrimination the nucleic acid has been passed through mutants of hemolysin (WO 2014/100481) or of lysenin (WO 2013/153359).

There is still a need for rapid and cheap nucleic acid (e.g. DNA or RNA) sequencing technologies across a wide range of applications. Existing technologies are slow and expensive mainly because they rely on amplification techniques to produce large volumes of nucleic acid and require a high quantity of specialist fluorescent chemicals for signal detection. Nanopore sensing has the potential to provide rapid and cheap nucleic acid sequencing by reducing the quantity of nucleotide and reagents required. However, the available nanopore-based solutions for nucleic acid sequencing are still far to be optimized.

Aerolysin, produced by Aeromonas species, is the founding member of a large superfamily that spans all of the kingdoms of life. Although it was the first *β* -PFT for which the X-ray structure of the soluble form was solved, the structure of the pore has remained elusive for long time. Aerolysin forms a heptameric beta-barrel in biological membranes. It is secreted as a monomer that binds to the outer membrane of susceptible cells. Upon binding, the monomers oligomerize to form a water-filled transmembrane channel that facilitates uncontrolled permeation of water, ions, and small organic molecules. Rapid discharge of vital molecules, such as ATP, dissipation of the membrane potential and ionic gradients, and irreversible osmotic swelling leading to rupture or lysis of the cell wall, frequently causing death of the host cell. This pore-forming property has been identified as a major mechanism by which protein toxins cause damage to cells.

Aerolysin is the most promising one for identifying the subtle difference of detected molecules because of its unique structure profile. Cao C. et al. (Nat Nanotechnol. 2016 Apr 25. doi: 10.1038/nnano.2016.66) demonstrated the ability of aerolysin nanopore to resolve at high resolution individual short oligonucleotides that are 2 to 10 bases long without any extra chemicals or modifications, useful for single-molecule analysis of oligonucleotides.

Additionally, Cao C. et al. (Nature Communications volume 9, Article number: 2823, 2018) described nanopore experimental results and molecular simulations based on an aerolysin structural model to map the sensing spots for ssDNA translocation. Computational and experimental results revealed two critical sensing spots (R220, K238) generating two constriction points along the pore lumen. Taking advantage of the sensing spots, all four nucleobases, cytosine methylation and oxidation of guanine can be clearly identified in a mixture sample. However, no rational mutagenesis design of aerolysin to improve its sensing capabilities are described therein.

US2022/0127310A1 disclosed a range of aerolysin nanopore improved mutants and their use for sequencing DNA. However, the efficiency of the disclosed mutants is limited in terms of base-calling accuracy recalling due to the influence from the bases flanking the base being read. There is thus a need to search for even better pore candidates that are capable of reading nucleic acids with higher accuracy.

It is further sought to improve the translocation of the analyte through the aerolysin nanopore, thus increasing the efficiency of the method.

### Summary of the invention

The present invention is based at least in part on a series of aerolysin mutants that have been rationally designed and studied, using molecular modelling and simulation based on recent aerolysin structures and models, in order to alter the interaction between an aerolysin monomer and an analyte such as a polynucleotide, polypeptide or small molecules such as ions.

The presently provided mutant is characterized by a constriction site having a smaller diameter and a smaller height, compared to prior art aerolysin monomers. In preferred aspects, the aerolysin monomer also advantageously comprises only one single constriction site. These features enable an absolutely single nucleotide resolution of the reading site.

The aerolysin mutants of the present invention are based on
- SEQ ID NO: 1 (native, unmodified aerolysin monomer polypeptide from *Aeromonas hydrophila*) or homologs or paralogs thereof of SEQ ID NO:2 to 7, or
- SEQ ID NO:8 (mature aerolysin monomer derived from SEQ ID NO:1, in which the C-term propeptide is removed) or homologs or paralogs thereof of SEQ ID NO: 10 to 15,
in which specific modifications of the pore region aim at improving the reading resolution of the pore.

The aerolysin mutants of the present invention at least bear one mutation in position 238, such as described below (herein designated as the first mutation) and preferably also a mutation in position 242, such as described below (herein designated as the second mutation).

More specifically, in a first embodiment, the invention relates to mutants of any one of SEQ ID NO:1, 2, 4, 6 to 8, 10, 12, 14 and 15, which at least bear one mutation at position 238 selected among K238A/S/G/D and mutants of any one of SEQ ID NO:3 and 11 which at least bear one mutation at position 238 selected among V238A/S/G/D The mutation in position 238 significantly increases the sensing accuracy of the aerolysin sequence, when used to form a pore in a sensing method.

In a second embodiment, the invention provides mutants of any one of SEQ ID NO:1, 2, 4, 6 to 8, 10, 12, 14 and 15 which at least bear one mutation at position 238 selected among K238A/S/G/Q/W/E/D/N in combination with one mutation at position 242 selected among K242W/A, mutants of any one of SEQ ID NO:3 and 11 which at least bear one mutation at position 238 selected among V238A/S/G/Q/W/E/D/N in combination with one mutation at position 242 selected among N242W/A and mutants of any one of SEQ ID NO:5 and 13 which at least bear one mutation at position 242 selected among S242W/A. Of note, SEQ ID NO:5 to 13 already have S in position 238 and do not require a mutation in this position for the purpose of the present invention.

These mutations are responsible for narrowing the diameter of the constriction site at position R220, which is the most important among the four restriction sites of aerolysin nanopore for identification of nucleobases to be read. Pores comprising the above-described mutation in position 238 have improved nucleotide reading resolution. Furthermore, the residence time of an analyte in the R220 constriction site is increased, which makes it possible to evaluate the current blockade more accurately, when the mutant polypeptide is used in a sensing method as disclosed below. The resolution and accuracy are further improved when the mutation in position 238 is combined with a mutation in position 242, such as described herein.

In a preferred aspect, the invention provides mutants of any one of SEQ ID NO:1, 2, 4, 6 to 8, 10, 12, 14 and 15 which at least bear one mutation at position 238 selected among K238A/S/G/D in combination with one mutation at position 242 selected among K242W/A, mutants of any one of SEQ ID NO:3 and 11 which at least bear one mutation at position 238 selected among V238A/S/G/D in combination with one mutation at position 242 selected among N242W/A and mutants of any one of SEQ ID NO:5 and 13 which at least bear one mutation at position 242 selected among S242W/A.

In preferred aspects, the mutants of the present invention bear additional mutations, intended to engineer the other constrictions sites of the wild-type aerolysin, in order to reduce the influence of the bases neighbouring the base to be read.

In other preferred aspects, the mutants are optimized to facilitate translocation of the nucleic acids through the pore, by increasing the net positive charge of the pore.

In other preferred aspects the mutants of the present invention enable the use of higher voltage, leading to even more efficient and accurate sequencing processes.

Accordingly, provided herein in a first aspect is a polypeptide selected from the group consisting of
a) a polypeptide comprising a modified aerolysin amino acid sequence comprising a first amino acid substitution selected from the group consisting of K238A, K238S, K238G and K238D of the amino acid sequence of any one of SEQ ID NO:1, 2, 4, 6 to 8, 10, 12, 14 and 15 or a variant or fragment thereof, preferably SEQ ID NO: 1 or 8, more preferably SEQ ID NO:8(also referred to herein as "mutant aerolysin"); and
b) a polypeptide comprising a modified aerolysin amino acid sequence comprising a first amino acid substitution selected from the group consisting of V238A, V238S, V238G and V238D of the amino acid sequence of any one of SEQ ID NO:3 and 11 or a variant or fragment thereof

In a second aspect the invention provides a polypeptide selected from the group consisting of
a) a polypeptide comprising a modified aerolysin amino acid sequence comprising a first amino acid substitution selected from the group consisting of K238A, K238S, K238G, K238Q, K238W, K238E, K238D and K238N and a second amino acid substitution selected from the group consisting of K242W and K242A of the amino acid sequence of any one of SEQ ID NO:1, 2, 4, 6 to 8, 10, 12, 14 and 15 or a variant or fragment thereof, preferably SEQ ID NO:1 or 8, more preferably SEQ ID NO:8 (also referred to herein as "mutant aerolysin");
b) a polypeptide comprising a modified aerolysin amino acid sequence comprising a first amino acid substitution selected from the group consisting of V238A, V238S, V238G, V238Q, V238W, V238E, V238D and V238N and a second amino acid substitution selected from the group consisting of N242W and N242A of the amino acid sequence of any one of SEQ ID NO:3 and 11 or a variant or fragment thereof; and
c) a polypeptide comprising a modified aerolysin amino acid sequence comprising an amino acid substitution selected from the group consisting of S242W and S242A of the amino acid sequence of any one of SEQ ID NO:5 and 13 or a variant or fragment thereof.

A modified aerolysin polypeptide generally comprises, consists essentially of or consists of a modified aerolysin amino acid sequence as described herein.

When the polypeptide is a mutant of the amino acid of any one of SEQ ID NO:8 or 10 to 15, or a variant or fragment thereof, the polypeptide forms an aerolysin monomer that can form an aerolysin pore upon oligomerization with other monomers. Information on the structural features of the aerolysin monomer in all its states and the pore obtainable therefrom can be retrieved on the website http://www.uniprot.org/, access number P09167, and previously described in literature in e.g. Degiacomi M.T. et al., Nat Chem Biol. 2013 Oct;9(10):623-9. Thus, in a third aspect, the present invention provides an aerolysin pore comprising a polypeptide selected from:
a) a polypeptide comprising a modified aerolysin amino acid sequence comprising a first amino acid substitution selected from the group consisting of K238A, K238S, K238G and K238D of the amino acid sequence of any one of SEQ ID NO:8, 10, 12, 14 or 15 or a variant or fragment thereof, preferably SEQ ID NO:8; and
b) a polypeptide comprising a modified aerolysin amino acid sequence comprising a first amino acid substitution selected from the group consisting of V238A, V238S, V238G and V238D of the amino acid sequence of SEQ ID NO: 11 or a variant or fragment thereof;

In a fourth aspect, the present invention provides an aerolysin pore comprising a polypeptide selected from:
a) a polypeptide comprising a modified aerolysin amino acid sequence comprising a first amino acid substitution selected from the group consisting of K238A, K238S, K238G, K238Q, K238W, K238E, K238D and K238N and a second amino acid substitution selected from the group consisting of K242W and K242A of the amino acid sequence of any one of SEQ ID NO:8, 10, 12, 14 or 15 or a variant or fragment thereof, preferably SEQ ID NO:8;
b) a polypeptide comprising a modified aerolysin amino acid sequence comprising a first amino acid substitution selected from the group consisting of V238A, V238S, V238G, V238Q, V238W, V238E, V238D and V238N and a second amino acid substitution selected from the group consisting of N242W and N242A of the amino acid sequence of SEQ ID NO: 11 or a variant or fragment thereof; and
c) a polypeptide comprising a modified aerolysin amino acid sequence comprising an amino acid substitution selected from the group consisting of S242W and S242A of the amino acid sequence of SEQ ID NO: 13 or a variant or fragment thereof.

In fifth aspect, the present invention provides a construct comprising:
a) two or more covalently attached monomers derived from the mutant aerolysin polypeptide of the present invention;
b) a homo-oligomeric pore derived from said mutant aerolysin polypeptide comprising identical mutant monomers; or
c) a hetero-oligomeric pore derived from said mutant aerolysin polypeptide as described herein, wherein at least one of the monomers differs from the others.

In a sixth aspect, the present invention provides a method of sensing and/or characterising a target substrate comprising:
(a) contacting the target substrate with the mutant aerolysin pore of the invention so to allow the movement of the target substrate through said pore and a portion of the substrate interacts with said pore; and
(b) measuring a current passing through said pore, thereby sensing and/or characterising the target substrate. In some embodiments, steps (a) and (b) are carried out with a voltage applied across the pore. Preferably, the target substrate is a metal ion, an inorganic salt, a polymer, an amino acid, a peptide, a polypeptide, a protein, a nucleotide, an oligonucleotide, a polynucleotide, a dye, a bleach, a pharmaceutical, a diagnostic agent, a recreational drug, an explosive or an environmental pollutant.

In some embodiments, the substrate is a nucleic acid, and said contacting is controlled by handling enzyme so that the movement of the nucleic acid through the pore and a proportion of the nucleotides in the target sequence interacts with the pore. In one embodiment, the step of characterising a target polynucleotide comprises estimating the sequence of, or sequencing the target polynucleotide; and

In a seventh aspect, the present invention provides an apparatus for sensing a target substrate in a sample, comprising a mutant aerolysin pore according to the invention, wherein in some embodiments the target substrate is a nucleic acid sequence, further comprising a nucleic acid handling enzyme.

In an eighth aspect, the present invention also provides a system comprising a membrane having at least one mutant aerolysin pore according to the invention spanning across the membrane thickness.

In a ninth aspect, the present invention also relates to a nucleic acid encoding a polypeptide according to the invention, to a vector comprising such nucleic acid and to a host modified with such vector.

Further embodiments of the present invention are defined by the appended claims.

The above and other objects, features and advantages of the herein presented subject-matter will become more apparent from a study of the following description with reference to the attached figures showing some preferred aspects of said subject-matter.

### Description of the figures

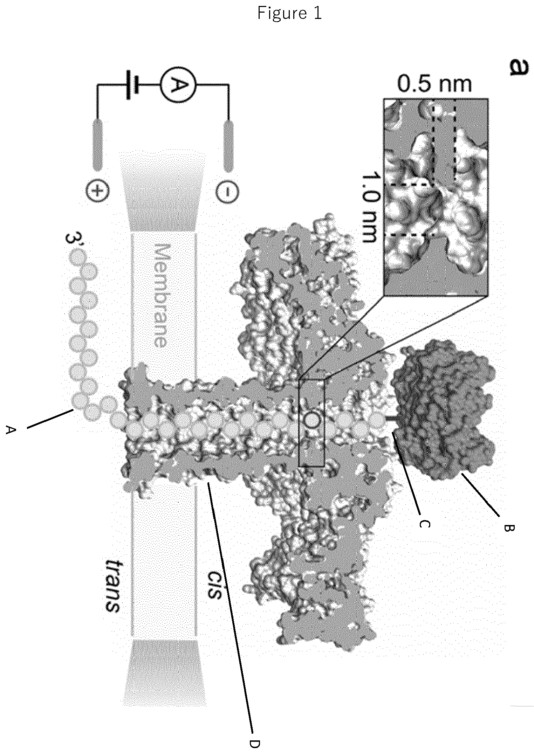

Figure 1 is a schematic diagram of streptavidin-biotin-ssDNA complex stalled in the aerolysin nanopore tested in Example 1 (D) and corresponding nucleotide current signature. The 5'-terminal of a ssDNA (A) specifically bound to a streptavidin (B) (PDB:1mk5 without biotin molecules) via a biotin linker (C). Structural model of aerolysin nanopore based on cryo-EM data12, 21 and mutated lysine at 238 position to alanine using the CHARMM-gui server22. The position order of each nucleotide in ssDNA was named from 5'-terminal. After apply a voltage of +140 mV, negatively charged ssDNA that carrying the whole complex is driven to thread through the pore from cis chamber until the bulky streptavidin prevents it from further translocation. Residual current was recorded as the ssDNA is immobilized within K238A aerolysin pore.

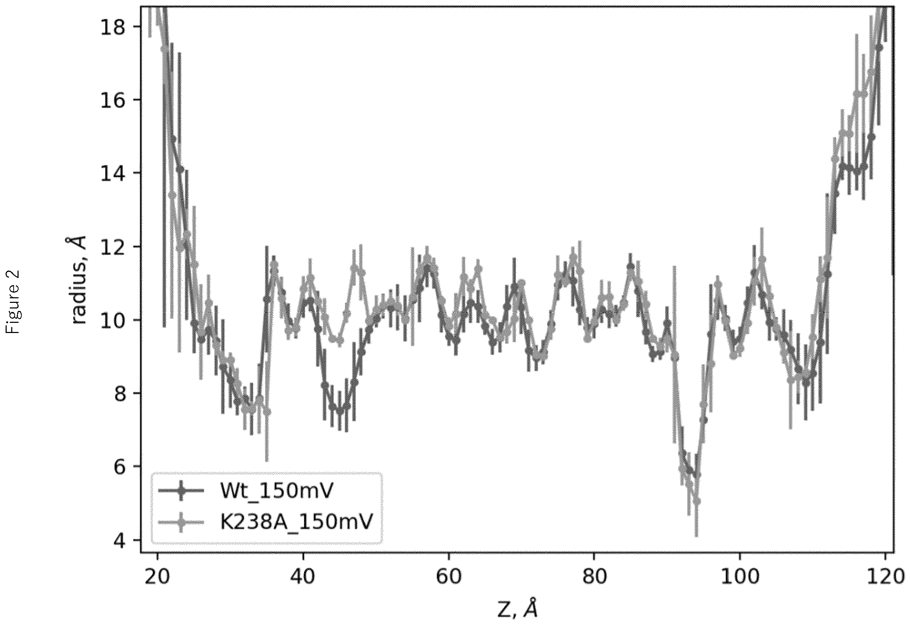

Figure 2 is a diagram of the average radial profile of the wt aerolysin pore (dark grey) and of the aerolysin pore tested in Example 1, having monomers of SEQ ID NO:16 (light grey). The average was taken over a 200 ns MD trajectory using the method described in Example 1. The error bars represent the standard deviations of the average radii due to the structure fluctuations of the channel.

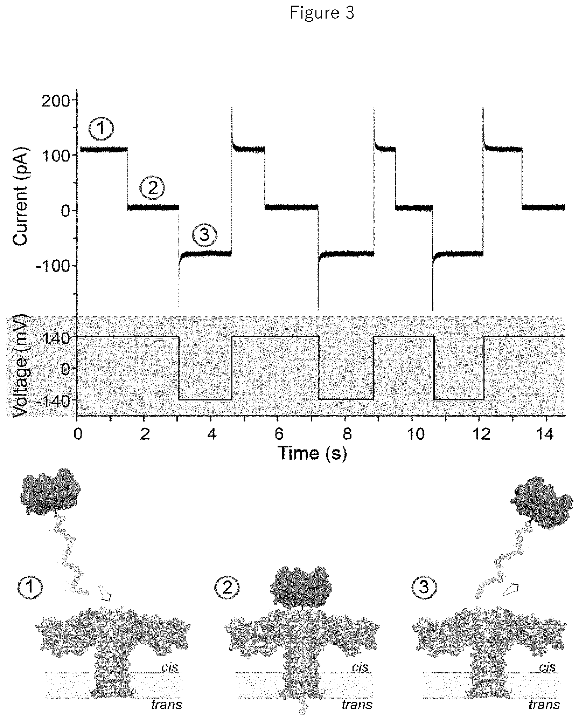

Figure 3 is an example of raw current trace recording. Upper: current and relative voltage (gray background) recording traces during a ssDNA-biotin-streptavidin experiment. The applied voltage was held at 140 mV until there was a spontaneous reduction in current corresponding to DNA entering the pore. The voltage was held at 140 mV for 1.5 s and then applied a -140 mV voltage to force ssDNA to get out of the pore and back into the cis chamber. Bottom: a schematic diagram of process happed during the recoding. Steps ① - ③ are corresponding to the three current state shown in the upper of figure.

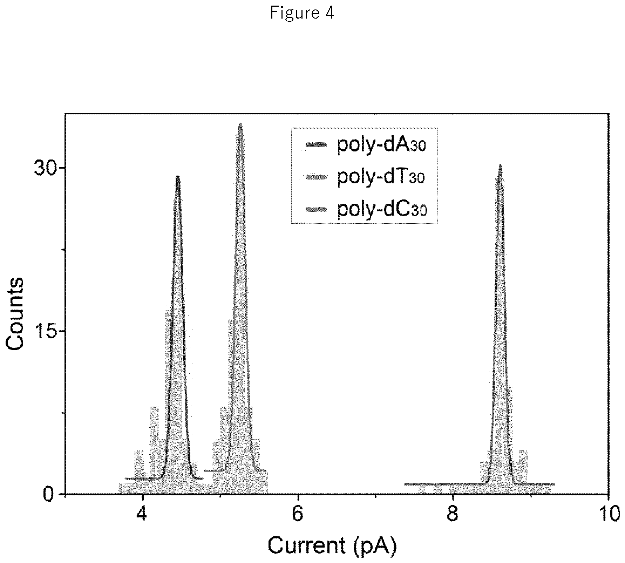

Figure 4 shows current histograms of dA30, dC30, and dT30 homopolymers and corresponding Gaussian fitting.

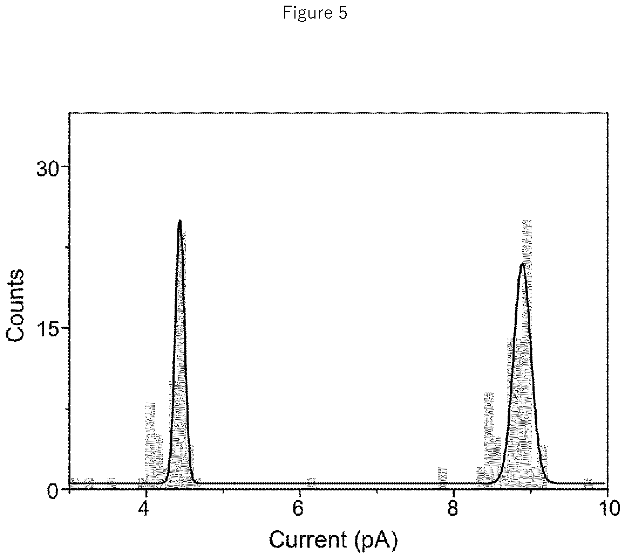

Figure 5 shows current histograms of mixture experiment of dA30 and dC30 and corresponding Gaussian fitting. The left peak is dA30 while the right is dC30.

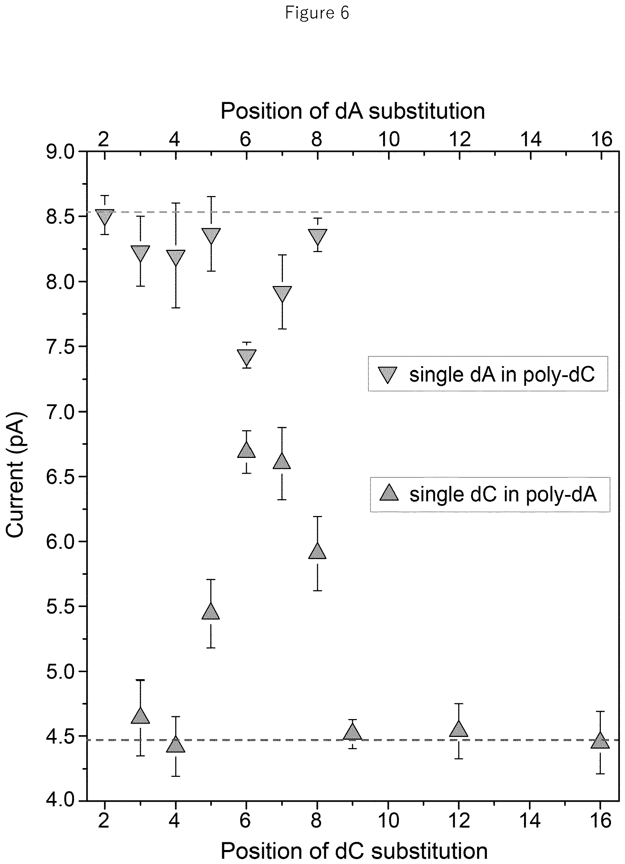

Figure 6 shows a plot of the mean current fitted value versus the position of single heteromeric substitution in homopolymer ssDNA, including a single dC substitution in a homopolymer of adenine (up triangle) and a single dA substitution in a homopolymer of cytosine (down triangle). For comparison, the mean current value of dA30 (lower dashed line) and dC30 (upper dashed line) are shown. Voltage conditions: +140mV.

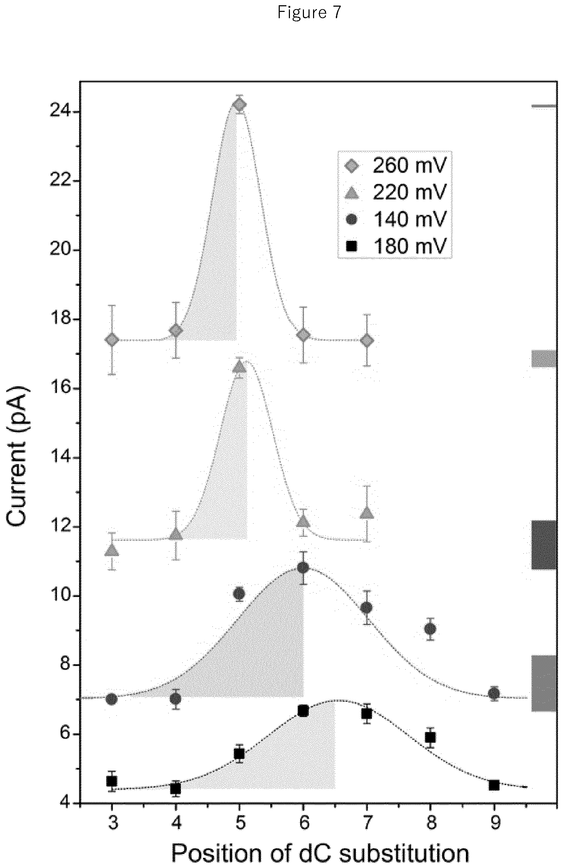

Figure 7 shows a plot of the current value versus the position of single heteromeric dC substitution in homopolymer ssDNA of adenine under different voltage conditions: 140 mV (dots), 180 mV (squares), 220 mV (triangles) and 260 mV (diamonds), respectively. For each voltage, the difference between the highest measured current value and the current value obtained with a homopolymer of cysteine (dC30) is represented for each voltage as a boxed in the right side of the plot (from bottom to top: 180mV, 140 mV, 220 mV and 260 mV).

### Detailed description of the invention

The subject matter herein described will be clarified by means of the following description. It is however to be understood that the subject matter described in this specification is not limited to the aspects described herein and depicted in the drawings; to the contrary, the scope of the subject-matter herein described is defined by the claims. Moreover, it is to be understood that the specific conditions or parameters described and/or shown in the following are not limiting of the subject matter herein described, and that the terminology used herein is for the purpose of describing particular aspects by way of example only and is not intended to be limiting.

Unless otherwise defined, technical and scientific terms used herein have the same meaning as commonly understood by one of ordinary skill in the art to which this invention belongs. Further, unless otherwise required by the context, singular terms shall include pluralities and plural terms shall include the singular. The methods and techniques of the present disclosure are generally performed according to conventional methods well known in the art and as described in various general and more specific references that are cited and discussed throughout the present specification unless otherwise indicated. Further, for the sake of clarity, the use of the term "about" is herein intended to encompass a variation of +/- 10% of a given value.

As used in the following and in the appended claims, the singular forms "a", "an" and "the" include plural referents unless the context clearly dictates otherwise. Also, the use of "or" means "and/or" unless stated otherwise. Similarly, "comprise", "comprises", "comprising", "include", "includes" and "including" are interchangeable and not intended to be limiting. It is to be further understood that where for the description of various embodiments use is made of the term "comprising", those skilled in the art will understand that in some specific instances, an embodiment can be alternatively described using language "consisting essentially of" or "consisting of."

### Mutant aerolysin polypeptide

The present invention provides mutant aerolysin polypeptides. Such mutant polypeptides are either mutants of any of SEQ ID NO:1 to 7, variants or fragments thereof, or mutants of any of SEQ ID NO:8 or 10 to 15, variants or fragments thereof. Mutants of SEQ ID NO:1 to 7 are nonmature polypeptides that cannot form pores. Mutants of SEQ ID NO:8 or 10 to 15 are the mature forms of the variants of SEQ ID NO:1 to 7, wherein the C-terminal propeptide has been removed. Such variants are monomer that retain the ability to form pores together with other aerolysin monomers. Variants of any one of SEQ ID NO:1 to 7 can be turned into monomers by maturation of the polypeptide. After maturation, such Methods for confirming the ability of mutant monomers to form pores are well-known in the art and are discussed in more detail below. Variants of any one of SEQ ID NO:1 to 7 can be turned into monomers by maturation of the polypeptide (removal of the C-terminal propeptide), thus acquiring their ability to form monomers.

The mutant monomers have an altered sensing accuracy. Pores comprising one or more of the mutant monomers of the present invention therefore also have improved sensing accuracy. Such pores are also characterized by a highly efficient analyte translocation. Furthermore, the pores of the invention enable the use of current with a higher voltage compared to pores typically used in the art, which makes it easier to discriminate between different nucleotides or amino acids and leads to more accurate sensing methods using such pores.

For the purpose of the present invention, it is essential that the mutant polypeptides have an amino acid selected from A, S, G, Q, W, E, D and N, preferably A, S, G, or D, more preferably A, S or G, , more preferably A or S, most preferably A in position 238 and an amino acid selected from W and A, most preferably A in position 242.
a)

In preferred aspect, the first amino acid substitution is selected from the group consisting of K238A, K238S, K238G and K238D or from the group consisting of V238A, V238S, V238G, and V238D, respectively. More preferably, it is selected from the group consisting of K238A, K238S and K238G, or from the group consisting of V238A, V238S and V238G, respectively. Even more preferably, it is selected from the group consisting of K238A and K238S or from the group consisting of V238A and V238S respectively. Most preferably it is K238A or V238A, respectively.

In another preferred aspect, the second amino acid substitution is K242A, N242A or S242A, respectively.

The first and the second mutation result in a narrower diameter of the constriction site in position R220.

Preferred polypeptides according to the present invention are polypeptides of SEQ ID NO:16, which is a polypeptide of SEQ ID NO:1, modified with the substitution K238A, and the polypeptide of SEQ ID NO:17, which is the polypeptide of SEQ ID NO:16, further modified with the substitution K242A.

In another preferred aspect, the mutant polypeptide or monomer comprises at least one additional mutation that contributes to the removal of other constriction sites in the monomer. Indeed, wild-type aerolysin comprises four constriction sites, at positions 282, 220, 238 and 242. The constriction site at position 220 is the constriction site having the narrowest diameter among the aerolysin constriction sites. It is thus the main constriction site of an aerolysin monomer for analytical purposes. As explained above, the mutants of the present invention are engineered to further reduce the diameter of the constriction site at position 220 (due to the first and second amino acid substitution). In order to further improve sensing accuracy, preferred mutants of the present invention are also engineered to increase the diameter or even eliminate the constriction sites in positions 282, 238 and 242. This results in improved accuracy, as it reduces the impact of secondary constriction sites on the signal. Thus, the mutant polypeptide or monomer comprises at least one of
- a third amino acid substitution selected from the group consisting of R282A, R282S, R282G, R282D, R282W, preferably selected from the group consisting of R282A and R282S, most preferably R282S when the sequence is selected from any one of SEQ ID NO:1,2, 4, 6 to 8, 10, 12, 14 and 15 or a variant or fragment thereof or a third amino acid substitution selected from P282A, P282S, P282G, P282D, P282W, preferably selected from the group consisting of P282A and P282S, most preferably P282S when the sequence is selected from SEQ ID NO:3 and 11 or a variant or fragment thereof;
- a fourth amino acid substitution selected from the group consisting of D216A, D216S, D216G, D216D and D216W, preferably selected from the group consisting of D216A and D216S, most preferably D216S when the sequence is selected from any one of SEQ ID NO:1, 2, 4, 7, 8, 10, 12 and 15 or a variant or fragment thereof or a fourth amino acid substitution selected from selected from the group consisting of V216A, V216S, V216G, V216D and V216W, preferably selected from the group consisting of V216A and V216S, most preferably V216S when the sequence is selected from SEQ ID NO: 3, 6, 11 or 14 or a variant or fragment thereof or a fourth amino acid substitution selected from selected from the group consisting of G216A and G216S, most preferably V216S when the sequence is selected from SEQ ID NO: 5 and 13 or a variant or fragment thereof;
- a fifth amino acid substitution selected from the group consisting of D222A, D222S, D222G and D222D, preferably selected from the group consisting of D222A and D222S, most preferably D222S when the sequence is selected from any one of SEQ ID NO:1,2, 4, 6 to 8, 10, 12, 14 and 15 or a variant or fragment thereof or a fifth amino acid substitution selected the group consisting of K222A, K222S, K222G and K222D, preferably selected from the group consisting of K222A and K222S, most preferably K222S when the sequence is selected from SEQ ID NO:3 or 11 or a variant or fragment thereof or a fifth amino acid substitution selected from the group consisting of N222A, N222S, N222G and N222D, preferably selected from the group consisting of N222A and N222S, most preferably N222S when the sequence is selected from any one of SEQ ID NO: 5 and 13 or a variant or fragment thereof;
- a sixth amino acid substitution selected from the group consisting of E258A, E258S, E258G, E258D and E258W, preferably selected from the group consisting of E258A and E258S, most preferably E258S when the sequence is selected from any one of SEQ ID NO:1, 2, 4, 6 to 8, 10, 12, 14 and 15 or a variant or fragment thereof or a sixth amino acid substitution selected from the group consisting of I258A, I258S, I258G, I258D and I258W, preferably selected from the group consisting of I258A and I258S, most preferably I258S when the sequence is selected from any one of SEQ ID NO: 3 and 11 or a variant or fragment thereof;
- a seventh amino acid substitution selected from the group consisting of E254A, E254S, E254G, E254D and E254W, preferably selected from the group consisting of E254A and E254S, most preferably E254S when the sequence is selected from any one of SEQ ID NO:1,2, 4, 5, 6 to 8, 10, 12, 13, 14 and 15 or a variant or fragment thereof or a seventh amino acid substitution selected from the group consisting of L254A, L254S, L254G, L254D and L254W, preferably selected from the group consisting of L254A and L254S, most preferably L254S when the sequence is selected from any one of SEQ ID NO: 3 and 11 or a variant or fragment thereof;
- an eighth amino acid substitution selected from the group consisting of K244A, K244S, K244G, K244D and K244W, preferably selected from the group consisting of K244A and K244S, most preferably K244S when the sequence is selected from any one of SEQ ID NO:1, 2, 4, 5, 6 to 8, 10, 12, 13, 14 and 15 or a variant or fragment thereof or an eighth amino acid substitution selected from the group consisting of F244A, F244S, F244G, F244D and F244W, preferably selected from the group consisting of F244A and F244S, most preferably F244S when the sequence is selected from any one of SEQ ID NO: 3 and 11 or a variant or fragment thereof;
- a ninth amino acid substitution selected from the group consisting of E252A, E252S, E252G, E252D and E252W, preferably selected from the group consisting of E252A and E252S, most preferably E252S when the sequence is selected from any one of SEQ ID NO:1, 2, 4, 6 to 8, 10, 12, 14 and 15 or a variant or fragment thereof or a ninth amino acid substitution selected from the group consisting of T252A, T252S, T252G, T252D and T252W, preferably selected from the group consisting of T252A and T252S, most preferably T252S when the sequence is selected from any one of SEQ ID NO: 3 and 11 or a variant or fragment thereof or a ninth amino acid substitution selected from the group consisting of V252A, V252S, V252G, V252D and V252W, preferably selected from the group consisting of V252A and V252S, most preferably V252S when the sequence is selected from any one of SEQ ID NO: 5 and 13 or a variant or fragment thereof; and
- a tenth amino acid substitution selected from the group consisting of K246S, K246A K246G, K246D and K246W, preferably selected from the group consisting of K246A and K246S, most preferably K246S when the sequence is selected from any one of SEQ ID NO:1, 2, 4, 5, 7, 8, 10, 12, 13, and 15 or a variant or fragment thereof or a tenth amino acid substitution selected from the group consisting of W246S, W246A W246G, W246D and W246W, preferably selected from the group consisting of W246A and W246S, most preferably W246S when the sequence is selected from any one of SEQ ID NO: 3 and 11 or a variant or fragment thereof or a tenth amino acid substitution selected from the group consisting of Q246S, Q246A Q246G, Q246D and Q246W, preferably selected from the group consisting of Q246A and Q246S, most preferably Q246S when the sequence is selected from any one of SEQ ID NO: 6 and 14 or a variant or fragment thereof.

In a preferred aspect, the polypeptides or monomers of the present invention comprises at least a first, a second and a third amino acid substitution as described above; preferably it comprises exactly such mutations. In a more preferred aspect, the polypeptides or monomers of the present invention comprises at least a first, a second, a third and a fourth amino acid substitution as described above; preferably it comprises exactly such mutations. In a more preferred aspect, the polypeptides or monomers of the present invention comprises at least a first, a second, a third, a fourth and a fifth amino acid substitution as described above; preferably it comprises exactly such mutations. In a more preferred aspect, the polypeptides or monomers of the present invention comprises at least a first, a second, a third, a fourth, a fifth and a sixth amino acid substitution as described above; preferably it comprises exactly such mutations. In a more preferred aspect, the polypeptides or monomers of the present invention comprises at least a first, a second, a third, a fourth, a fifth, a sixth and a seventh amino acid substitution as described above; preferably it comprises exactly such mutations. In a more preferred aspect, the polypeptides or monomers of the present invention comprises at least a first, a second, a third, a fourth, a fifth, a sixth, a seventh amino acid and an eighth amino acid substitution as described above; preferably it comprises exactly such mutations. In an even more preferred aspect, the polypeptides or monomers of the present invention comprises at least a first, a second, a third, a fourth, a fifth, a sixth, a seventh, an eighth and a ninth amino acid substitution as described above; preferably it comprises exactly such mutations. In a most preferred aspect, the polypeptides or monomers of the present invention comprises all of the amino acid substitution described above; preferably it comprises exactly such mutations. By the terms " it comprises exactly such mutations" it is meant that the polypeptide or monomer corresponds to any one of SEQ ID NO:1 to 8 or 10 to 15 (or a variant or fragment thereof), respectively, mutated to bear all of the recited mutations and only such recited mutations, in accordance with any of the above-described embodiments.

The constriction site at position 220 is preferably characterized by a diameter of at most 1.2 nm, more preferably at most 1.1 nm, most preferably at most 1.0 nm. In another preferred aspect of the invention, the constriction site at position 220 is characterized by a height of at most 0.7 nm, preferably at most 0.6 nm, most preferably at most 0.5 nm. For the sake of clarity, diameter and height as referred herein cannot be less than 0.1 nm in size.

The combination of the first and second mutations ultimately results in a narrowing of the 220 reading site enabling this site to read one single base, without interference of the neighbouring bases. Combination of all additional amino acid substitutions ultimately lead to a monomer having only one reading site, avoiding the interference of other sites on the signal. Thus, the accuracy is improved in such an extent that a single base reading is achieved, wherein the signal corresponds to only one base read by one single restriction site.

Such engineering of the monomer of is also advantageous in that it makes it possible to use currents with higher voltage when the monomers are used in a sensing method such as described below, thus further increasing the accuracy of the sensing method.

Furthermore, the above-mutations are tailored to improve the translocation of the analyte through the pore. This is achieved mainly be increasing the net positive charge of the pore. Said net charge is increased by e.g. introducing one or more positively charged amino acids and/or by neutralising one or more negative charged amino acids, for instance by substituting one or more negatively charged amino acids with one or more uncharged amino acids, non-polar amino acids and/or aromatic amino acids or by introducing one or more positive charged amino acids adjacent to one or more negatively charged amino acids.

In particular aspects, the pores constructed from the present mutant monomers easily capture and efficiently translocate at optimal rate (i.e., few nucleotides per millisecond, such as 2, 3, 4, 5, 6, 7, 8, 9, or 10 nucleotides per millisecond) nucleotides and polynucleotides. The same is true, *mutatis mutandis,* for other substrates such as peptides/polypeptides.

Importantly, the monomers of the present invention retain their ability to form a pore. As it will be evident to a person skilled in the relevant art, the ability of the monomer of the invention to form a pore, as for many other similar pore-forming polypeptides, derives from its structure and the presence of suitable, e.g. physiological, homo-hetero oligomerization conditions. In particular, the aerolysin monomers, both in the wild-type and in the mutated form, undergo a maturation/folding process that foresees several passages. Aerolysin is produced as an inactive precursor, proaerolysin, which contains a C-terminal peptide (CTP) required for folding into its soluble form. Proteolysis in the loop that connects the CTP to the main body allows aerolysin to oligomerize in a heptameric ring-like complex that inserts into the target membrane to form the pore. It is therefore herein tacitly understood that, when referring to a formed pore, the monomer of the invention comprises, consists of or substantially consists of a polypeptide having the sequence shown in any one of SEQ ID NO: 8 or 10 to 15, i.e. the mature aerolysin monomer without an N-terminal signal peptide and without a C-terminal propeptide, and which substantially differs from the sequence shown in SEQ ID NO: 1 in the C-terminal domain. "Substantially" herein means that, upon alignment of SEQ ID NO: 1 with a sequence comprising SEQ ID NO: 8, no more than five consecutive amino acid residues in the CTP must be equal.

The ability of the monomer to interact with a substrate such as a polynucleotide can be determined using methods that are well-known in the art. The monomer may interact with a substrate in any way, e.g. by non-covalent interactions, such as hydrophobic interactions, hydrogen bonding, Van der Waal's forces or electrostatic forces. For instance, the ability of the region to bind to a polynucleotide can be measured using a conventional binding assay.

Suitable assays include, but are not limited to, fluorescence-based binding assays, nuclear magnetic resonance (NMR), Isothermal Titration Calorimetry (ITC) or Electron spin resonance (ESR) spectroscopy.

Modifications of protein nanopores that alter their ability to interact with a polynucleotide, in particular improve their ability to capture and/or recognise or discriminate polynucleotides, are well documented in the art. For instance, such modifications are disclosed in WO 2010/034018 and WO 2010/055307. Similar modifications can be made to the aerolysin monomer in accordance with this invention.

In addition to the specific mutations discussed above, the variant may include other mutations. These mutations do not necessarily enhance the ability of the monomer to interact with an analyte substrate such as a polynucleotide. The mutations may facilitate, for example, expression and/or purification. Over the entire length of the amino acid sequence of any one of SEQ ID NO: 1 to 8 and 10 to 15, a variant will preferably be at least 50% homologous to that sequence based on amino acid identity. More preferably, the variant may be at least 55%, at least 60%, at least 65%, at least 70%, at least 75%, at least 80%, at least 85%, at least 90% and more preferably at least 95%, 97% or 99% homologous based on amino acid identity to the amino acid sequence of any one of SEQ ID NO: 1 to 8 and 10 to 15 over the entire sequence. There may be at least 80%, for example at least 85%, 90% or 95%, amino acid identity over a stretch of 100 or more, for example 125, 150, 175 or 200 or more, contiguous amino acids ("hard homology").

Standard methods in the art may be used to determine homology. For example, the UWGCG Package provides the BESTFIT program which can be used to calculate homology, for example used on its default settings (Devereux et al (1984) Nucleic Acids Research 12, p387-395). The PILEUP and BLAST algorithms can be used to calculate homology or line up sequences (such as identifying equivalent residues or corresponding sequences (typically on their default settings)). Software for performing BLAST analyses is publicly available through the National Center for Biotechnology Information (http://www.ncbi.nlm.nih.gov/).

Amino acid substitutions may be made to any one of the amino acid sequence of SEQ ID NO: 1 to 8 or 10 to 15, in addition to those discussed above, for example up to 1, 2, 3, 4, 5, 10, 20, 30 or even more substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in Table 1 below. Where amino acids have similar polarity, this can also be determined by reference to the hydropathy scale for amino acid side chains in Table 2.

**Table 1 - Chemical properties of amino acids**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gin | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| lie | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

**Table 2 - Hydropathy scale**

| Side Chain | Hydropathy |
|---|---|
| lie | 4.5 |
| Val | 4.2 |
| Leu | 3.8 |
| Phe | 2.8 |
| Cys | 2.5 |
| Met | 1.9 |
| Ala | 1.8 |
| Gly | -0.4 |
| Thr | -0.7 |
| Ser | -0.8 |
| Trp | -0.9 |
| Tyr | -1.3 |
| Pro | -1.6 |
| His | -3.2 |
| Glu | -3.5 |
| Gin | -3.5 |
| Asp | -3.5 |
| Asn | -3.5 |
| Lys | -3.9 |
| Arg | -4.5 |

A variant may comprise one or more substitutions beyond those specified above, in which amino acids are replaced with those at the corresponding position(s) in homologues and paralogues of aerolysin.

One or more amino acid residues of the amino acid sequence of any one of SEQ ID NO: 1 to 8 and 10 to 15 may additionally be deleted from the variants described above. Up to 1, 2, 3, 4, 5, 10, 20 or 30 residues may be deleted, or more.

Variants may include fragments of any one of SEQ ID NO: 1 to 8 or 10 to 15. Such fragments retain pore forming activity. This may be assayed as described above. Fragments may be at least 50, 100, 150, 200 or 250 amino acids in length. Such fragments may be used to produce the pores of the invention.

A fragment more preferably comprises the region from about position 216 to about position 282 of any one of SEQ ID NO: 1 to 8 or 10 to 15, preferably of SEQ ID NO:1 or SEQ ID NO:8, which is modified in accordance with the invention.

One or more amino acids may be alternatively or additionally added to the mutant polypeptides or monomers described above. An extension may be provided at the amino terminal or carboxy terminal of the amino acid sequence of the variant of any one of SEQ ID NO: 1 to 8 or 10 to 15, preferably SEQ ID NO:1 or SEQ ID NO:8, including a fragment thereof. The extension may be quite short, for example from 1 to 10 amino acids in length. Alternatively, the extension may be longer, for example up to 50 or 100 amino acids. A carrier protein may be fused to an amino acid sequence according to the invention. Other fusion proteins are discussed in more detail below.

In addition to the modifications of the invention, a variant of any one of SEQ ID NO: 1 to 8 or 10 to 15, preferably SEQ ID NO:1 or 8, may include one or more additional modifications, such as substitutions, additions or deletions. These modifications are preferably located in the stretches in the variant that correspond to from about position 1 to about position 206 and from about position 291 to about position 493 of any one of SEQ ID NO: 1 to 8 or 10 to 15 (i.e. outside of the region modified in accordance with the invention).

The mutant polypeptide or monomer may be modified to assist their identification or purification, for example by the addition of histidine residues (a "his tag"), aspartic acid residues (an "asp tag"), a streptavidin tag or a flag tag, or by the addition of a signal sequence to promote their secretion from a cell where the polypeptide does not naturally contain such a sequence. An alternative to introducing a genetic tag is to chemically react a tag onto a native or engineered position on the pore. An example of this would be to react a gel-shift reagent to a cysteine engineered on the outside of the pore. This has been demonstrated in a similar context as a method for separating hemolysin hetero-oligomers (Chem Biol. 1997 Jul;4(7):497-505).

The mutant polypeptide or monomer may be labelled with a revealing label. The revealing label may be any suitable label which allows the pore to be detected. Suitable labels include, but are not limited to, fluorescent molecules, radioisotopes, enzymes, antibodies, antigens, polynucleotides, polyethylene glycols (PEGs), peptides and ligands such as biotin.

The mutant polypeptide or monomer may also be produced using D-amino acids. For instance, the mutant monomer may comprise a mixture of L-amino acids and D-amino acids. This is conventional in the art for producing such proteins or peptides.

The mutant polypeptide or monomer contains one or more specific modifications to facilitate interaction with a substrate, such as a polynucleotide, polypeptide or small molecule analyte. The mutant polypeptide or monomer may also contain other non-specific modifications as long as they do not interfere with pore formation. A number of non-specific side chain modifications are known in the art and may be made to the side chains of the mutant monomer.

Such modifications include, for example, reductive alkylation of amino acids by reaction with an aldehyde followed by reduction with NaBH4, amidination with methylacetimidate or acylation with acetic anhydride.

The mutant polypeptide or monomer can be produced using standard methods known in the art. The monomer may be made synthetically or by recombinant means. For example, the monomer may be synthesized by in vitro translation and transcription (IVTT). Suitable methods for producing pore monomers are discussed in International Applications WO 2010/004273, WO 2010/004265 or WO 2010/086603. Methods for inserting pores into membranes are discussed below.

A mutant polypeptide or monomer of the invention may be isolated, substantially isolated, purified or substantially purified. A mutant polypeptide or monomer of the invention is isolated or purified if it is completely free of any other components, such as lipids. A mutant polypeptide or monomer is substantially isolated if it is mixed with carriers or diluents which will not interfere with its intended use. For instance, a mutant polypeptide or monomer is substantially isolated or substantially purified if it is present in a form that comprises less than 10%, less than 5%, less than 2% or less than 1% of other components, such as lipids.

Polynucleotide sequences encoding a mutant polypeptide or monomer may be derived and replicated using standard methods in the art. Such sequences are discussed in more detail below. Polynucleotide sequences encoding a mutant polypeptide or monomer may be expressed in a bacterial host cell using standard techniques in the art. The mutant polypeptide or monomer may be produced in a cell by in situ expression of the polypeptide from a recombinant expression vector. The expression vector optionally carries an inducible promoter to control the expression of the polypeptide.

A mutant polypeptide or monomer may be produced in large scale following purification by e.g. any protein liquid chromatography system from pore producing organisms or after recombinant expression as described below. Typical protein liquid chromatography systems include FPLC, AKTA systems, the Bio-Cad system, the Bio-Rad BioLogic system and the Gilson FIPLC system.

In some embodiments, the mutant polypeptide or monomer is chemically modified. The mutant polypeptide or monomer can be chemically modified in any way and at any site. The mutant polypeptide or monomer is preferably chemically modified by attachment of a molecule to one or more cysteines (cysteine linkage), attachment of a molecule to one or more lysines, attachment of a molecule to one or more non-natural amino acids, enzyme modification of an epitope or modification of a terminus. Suitable methods for carrying out such modifications are well-known in the art. Suitable non-natural amino acids include, but are not limited to, 4-azido-L-phenylalanine (Faz).

The mutant polypeptide or monomer may be chemically modified by the attachment of any molecule. For instance, the mutant polypeptide or monomer may be chemically modified by attachment of a polyethylene glycol (PEG), a nucleic acid, such as DNA, a dye, a fluorophore or a chromophore. In some embodiments, the mutant polypeptide or monomer is chemically modified with a molecular adaptor that facilitates the interaction between a pore comprising the polypeptide or monomer and a target substrate analyte, particularly a target nucleotide or target polynucleotide. The presence of the adaptor improves the host-guest chemistry of the pore and the nucleotide or polynucleotide and thereby improves the sequencing ability of pores formed from the mutant polypeptide or monomer. The principles of host-guest chemistry are well-known in the art. The adaptor has an effect on the physical or chemical properties of the pore that improves its interaction with the nucleotide or polynucleotide. The adaptor may alter the charge of the barrel or channel of the pore or specifically interact with or bind to the nucleotide or polynucleotide thereby facilitating its interaction with the pore.

The molecular adaptor is preferably a cyclic molecule, for example a cyclodextrin, a species that is capable of hybridization, a DNA binder or interchelator, a peptide or peptide analogue, a synthetic polymer, an aromatic planar molecule, a small positively-charged molecule or a small molecule capable of hydrogen-bonding.

The adaptor may be cyclic. A cyclic adaptor preferably has the same symmetry as the pore. The adaptor typically interacts with the substrate analyte, nucleotide or polynucleotide via host-guest chemistry. The adaptor is typically capable of interacting with the nucleotide or polynucleotide. The adaptor comprises one or more chemical groups that are capable of interacting with the nucleotide or polynucleotide. The one or more chemical groups preferably interact with the nucleotide or polynucleotide by non-covalent interactions, such as hydrophobic interactions, hydrogen bonding, Van der Waal's forces, p-cation interactions and/or electrostatic forces.

### Polynucleotide binding proteins

In certain embodiments, polynucleotide binding proteins may be covalently attached to the mutant monomer. The protein can be covalently attached to the pore using any method known in the art. The monomer and protein may be chemically fused or genetically fused. The monomer and protein are genetically fused if the whole construct is expressed from a single polynucleotide sequence. Genetic fusion of a pore to a polynucleotide binding protein is discussed in e.g. International Application WO 2010/004265.

If the polynucleotide binding protein is attached via cysteine linkage, the one or more cysteines have preferably been introduced to the mutant by substitution. Such substitutions are typically made in loop regions which have low conservation amongst homologues indicating that mutations or insertions may be tolerated. They are therefore suitable for attaching a polynucleotide binding protein. Such substitutions are typically made in residues 1 to 206 and 291 to 493 of any one of SEQ ID NO: 1 to 8 or 10 to 15.

The polynucleotide binding protein may be attached directly to the mutant monomer or via one or more linkers. The polynucleotide binding protein may be attached to the mutant monomer using the hybridization linkers described e.g. in International Application WO 2010/086602. Alternatively, peptide linkers may be used. Peptide linkers are amino acid sequences. The length, flexibility and hydrophilicity of the peptide linker are typically designed such that it does not to disturb the functions of the monomer and molecule. Preferred flexible peptide linkers are stretches of 2 to 20, such as 4, 6, 8, 10 or 16 amino acids. More preferred flexible linkers include (SG)1, (SG)2, (SG)3, (SG)4 and the like, wherein S is serine and G is glycine. Preferred rigid linkers are stretches of 2 to 30, such as 4, 6, 8, 16 or 24, proline amino acids.

### Making mutant aerolysin monomers

The invention also provides a method of improving the ability of an aerolysin monomer comprising any one of the sequences shown in SEQ ID NO: 8 or 10 to 15 to characterise an analyte such as a polynucleotide or peptide/polypeptide. The method comprises mutating any one of SEQ ID NO: 8 or 10 to 15 by substituting amino acid such as described above.

Any of the embodiments discussed above with reference to the mutant aerolysin monomers and below with reference to characterising polynucleotides/polypeptides equally apply to this method of the invention.

### Constructs

The invention also provides a construct comprising two or more covalently attached monomers derived from aerolysin wherein at least one of the monomers is a mutant aerolysin monomer of the invention. The construct of the invention retains its ability to form a pore. One or more constructs of the invention may be used to form pores for characterising a target substrate.

One or more constructs of the invention may be used to form pores for characterising a target polynucleotide, such as sequencing a target polynucleotide. The construct may comprise 2, 3, 4, 5, 6, 7 or more monomers, most preferably 7 monomers. The two or more monomers may be the same or different. At least one monomer in the construct is a mutant monomer of the invention. The other monomers in the construct do not have to be mutant monomers of the invention. For instance, at least one monomer may comprise the sequence shown in any one of SEQ ID NO: 8 or 10 to 15 or a variant or fragment thereof, preferably SEQ ID NO:8 or a variant or fragment thereof, preferably any one of SEQ ID NO: 8 to 15, most preferably SEQ ID NO:8.

Alternatively, at least one monomer may comprise a variant of any one of SEQ ID NO: 8 or 10 to 15, which is at least 50% homologous to any one of SEQ ID NO: 8 or 10 to 15 over its entire sequence based on amino acid identity, but does not include any of the specific mutations required by the mutant monomers of the invention. More preferably, the variant may be at least 55%, at least 60%, at least 65%, at least 70%, at least 75%, at least 80%, at least 85%, at least 90% and more preferably at least 95%, 97% or 99% homologous based on amino acid identity to the amino acid sequence of any one of SEQ ID NO: 8 or 10 to 15 over the entire sequence. The variant may be a fragment or any other variant discussed above.

Any or all of the monomers in the construct may be a mutant monomer of the invention. The mutant monomers may be the same or different. In a more preferred embodiment, the construct comprises seven monomers and at least one of the monomers is a mutant monomer according to the invention.

The monomers may be genetically fused. Monomers are genetically fused if the whole construct is expressed from a single polynucleotide sequence. The coding sequences of the monomers may be combined in any way to form a single polynucleotide sequence encoding the construct. Genetic fusion is discussed e.g. in International Application WO 2010/004265.

The monomers may be genetically fused in any configuration. The monomers may be fused via their terminal amino acids. For instance, the amino terminus of the one monomer may be fused to the carboxy terminus of another monomer.

The two or more monomers may be genetically fused directly together. The monomers are preferably genetically fused using a linker. The linker may be designed to constrain or facilitate the mobility of the monomers. Preferred linkers are amino acid sequences (i.e. peptide linkers).

In another embodiment, the monomers are chemically fused. Monomers are chemically fused if they are chemically attached, for instance via a chemical crosslinker. Any of the chemical crosslinkers discussed above may be used. The linker may be attached to one or more cysteine residues or non-natural amino acids, such as Faz, introduced into a mutant monomer. Alternatively, the linker may be attached to a terminus of one of the monomers in the construct.

If a construct contains different monomers, crosslinkage of monomers to themselves may be prevented by keeping the concentration of linker in a vast excess of the monomers. Alternatively, a "lock and key" arrangement may be used in which two linkers are used. Only one end of each linker may react together to form a longer linker and the other ends of the linker each react with a different monomers. Such linkers are described for instance in International Application WO 2010/086602.

The invention also provides a method of producing a construct of the invention. The method comprises covalently attaching at least one mutant aerolysin monomer of the invention to one or more monomers derived from aerolysin. Any of the embodiments discussed above with reference to the construct of the invention equally apply to the methods of producing the constructs.

### Polynucleotides

The present invention also provides polynucleotide sequences which encode a mutant polypeptide or monomer of the invention. The mutant polypeptide or monomer may be any of those discussed above. The polynucleotide sequence preferably comprises a sequence at least 50%, 60%, 70%, 80%, 90% or 95% homologous based on nucleotide identity to the sequence of SEQ ID NO: 9 over the entire sequence. There may be at least 80%, for example at least 85%, 90% or 95% nucleotide identity over a stretch of 600 or more contiguous nucleotides ("hard homology"). Homology may be calculated as described above. The polynucleotide sequence may comprise a sequence that differs from SEQ ID NO: 9 on the basis of the degeneracy of the genetic code.

The present invention also provides polynucleotide sequences which encode any of the genetically fused constructs of the invention, preferably two or more sequences encoding any of the genetically fused constructs of the invention or variants thereof as described above.

Polynucleotide sequences may be derived and replicated using standard methods in the art. Chromosomal DNA encoding wild-type aerolysin may be extracted from a pore producing organism, such as *Aeromonas hydrophi*/*a.* The gene encoding the pore monomer may be amplified using PCR involving specific primers. The amplified sequence may then undergo site-directed mutagenesis. Suitable methods of site-directed mutagenesis are known in the art and include, for example, combine chain reaction. Polynucleotides encoding a construct of the invention can be made using well-known techniques, such as those described in Sambrook, J. and Russell, D. (2001). Molecular Cloning: A Laboratory Manual, 3rd Edition. Cold Spring Harbor Laboratory Press, Cold Spring Harbor, NY.

The resulting polynucleotide sequence may then be incorporated into a recombinant replicable vector such as a cloning vector. The vector may be used to replicate the polynucleotide in a compatible host cell. Thus, polynucleotide sequences may be made by introducing a polynucleotide into a replicable vector, introducing the vector into a compatible host cell, and growing the host cell under conditions which bring about replication of the vector. The vector may be recovered from the host cell. Suitable host cells for cloning of polynucleotides are known in the art and described in more detail below.

The polynucleotide sequence may be cloned into suitable expression vector. In an expression vector, the polynucleotide sequence is typically operably linked to a control sequence which is capable of providing for the expression of the coding sequence by the host cell. Such expression vectors can be used to express a pore subunit.

The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences. Multiple copies of the same or different polynucleotide sequences may be introduced into the vector.

The expression vector may then be introduced into a suitable host cell. Thus, a mutant monomer or construct of the invention can be produced by inserting a polynucleotide sequence into an expression vector, introducing the vector into a compatible bacterial host cell, and growing the host cell under conditions which bring about expression of the polynucleotide sequence. The recombinantly-expressed monomer or construct may self-assemble into a pore in the host cell membrane. Alternatively, the recombinant pore produced in this manner may be removed from the host cell and e.g. inserted into another membrane. When producing pores comprising at least two different subunits, the different subunits may be expressed separately in different host cells as described above, removed from the host cells and assembled into a pore in a separate membrane.

The vectors may be for example plasmids, virus or phage vectors provided with an origin of replication, optionally a promoter for the expression of the said polynucleotide sequence and optionally a regulator of the promoter. The vectors may contain one or more selectable marker genes, for example a tetracycline resistance gene. Promoters and other expression regulation signals may be selected to be compatible with the host cell for which the expression vector is designed. A T7, trc, lac, ara or promoter is typically used.

The host cell typically expresses the pore subunit at a high level. Host cells transformed with a polynucleotide sequence will be chosen to be compatible with the expression vector used to transform the cell. The host cell is typically but not exclusively bacterial cells and preferably Escherichia coli. Host cells comprising an expression vector as described above are also contemplated in the present invention.

Examples of polynucleotides according to the invention include SEQ ID NO:18 and 19, which are both based on SEQ ID NO:9 with the mutation K238A (SEQ ID NO:18) and K238 and K242A (SEQ ID NO:19). These sequences do not include the C-terminal peptide and therefore encode the mature sequences SEQ ID NO:16 and 17, respectively.

### Pores

The invention also provides various pores. The pores of the invention are ideal for characterising substrate analytes. The pores of the invention are especially (but not exclusively) ideal for characterising, such as sequencing, polynucleotides because they might discriminate between different nucleotides with a high degree of sensitivity. The same is true, *mutatis mutandis,* for peptides and polypeptides. The pores can be used to characterise nucleic acids, such as DNA and RNA, including sequencing the nucleic acid and identifying single base changes. The pores of the invention could even distinguish between methylated and unmethylated nucleotides. The base resolution of pores of the invention is surprisingly high. The pores show almost complete separation of all four DNA nucleotides. The pores can be further used to discriminate between deoxycytidine monophosphate (dCMP) and methyl-dCMP based on the dwell time in the pore and the current flowing through the pore. Alternatively, the pores can be used to characterise polypeptides, including sequencing the amino acid sequence and identifying single amino acid changes, depending on the needs.

The pores of the invention can also discriminate between different nucleotides under a range of conditions. In particular, the pores will discriminate between nucleotides under conditions that are favourable to the characterisation, such as sequencing, of polynucleotides. The extent to which the pores of the invention can discriminate between different nucleotides can be controlled by altering the applied potential, the salt concentration, the buffer, the temperature and/or the presence of additives, such as urea, betaine and DTT. This allows the function of the pores to be fine-tuned, particularly when sequencing. This is discussed in more detail below.

The pores of the invention may also be used to identify polynucleotide polymers from the interaction with one or more monomers rather than on a nucleotide by nucleotide basis. A pore of the invention may be isolated, substantially isolated, purified or substantially purified. A pore of the invention is isolated or purified if it is completely free of any other components, such as lipids or other pores. A pore is substantially isolated if it is mixed with carriers or diluents which will not interfere with its intended use. For instance, a pore is substantially isolated or substantially purified if it is present in a form that comprises less than 10%, less than 5%, less than 2% or less than 1% of other components, such as lipids or other pores. Alternatively, a pore of the invention may be present in a lipid bilayer.

A pore of the invention may be present as an individual or single pore. Alternatively, a pore of the invention may be present in a homologous or heterologous population or plurality of two or more pores.

### Homo-oligomeric pores

The invention also provides a homo-oligomeric pore derived from aerolysin comprising identical mutant monomers of the invention. The monomers are identical in terms of their amino acid sequence. The homo-oligomeric pore of the invention is ideal for characterising, such as sequencing, polynucleotides. The homo-oligomeric pore of the invention may have any of the advantages discussed above.

The homo-oligomeric pore may contain any number of mutant monomers. The pore typically comprises two or more mutant monomers. One or more of the mutant monomers is preferably chemically modified as discussed above. In other words, one or more of the monomers being chemically modified (and the others not being chemically modified) does not prevent the pore from being homo-oligomeric as long as the amino acid sequence of each of the monomers is identical.

### Hetero-oligomeric pores

The invention also provides a hetero-oligomeric pore derived from aerolysin comprising at least one mutant monomer of the invention, wherein at least one of the monomers differs from the others. The monomer differs from the others in terms of its amino acid sequence. The hetero-oligomeric pore of the invention is ideal for characterising, such as sequencing, polynucleotides or polypeptides. Hetero-oligomeric pores can be made using methods known in the art (e.g. Protein Sci. 2002 Jul;l 1(7): 1813-24).

The hetero-oligomeric pore contains sufficient monomers to form the pore. The monomers may be of any type. The pore typically comprises seven monomers. The pore may comprise at least one monomer comprising the sequence shown in any one of SEQ ID NO: 8 or 10 to 15 or a variant or fragment thereof, which does not necessarily have a mutation required by the mutant monomers of the invention. In this embodiment, the remaining monomers are preferably mutant monomers of the invention.

In some embodiments, the pore comprises (a) one mutant monomer of the invention and (b) a sufficient number of identical monomers to form the pore, wherein the mutant monomer in (a) is different from the identical monomers in (b). The identical monomers in (b) preferably comprise the sequence shown in any one of SEQ ID NO: 8 or 10 to 15, a variant or a fragment thereof, which does not have a mutation required by the mutant monomers of the invention.

A hetero-oligomeric pore of the invention may comprise only one mutant aerolysin monomer of the invention. In another embodiment, all of the monomers in the hetero-oligomeric pore are mutant monomers of the invention and at least one of them differs from the others. In any of the embodiments discussed above, one or more of the mutant monomers may be chemically modified as discussed above. The presence of a chemical modification on one monomer does not result in the pore being hetero-oligomeric. The amino acid sequence of at least one monomer must differ from the sequence(s) of the other monomers. Methods for making pores are discussed in more detail below.

### Construct-containing pores

The invention also provides a pore comprising at least one construct of the invention. A construct of the invention comprises two or more covalently attached monomers derived from aerolysin, wherein at least one of the monomers is a mutant aerolysin monomer of the invention. In other words, a construct must contain more than one monomer. At least two of the monomers in the pore are in the form of a construct of the invention. The monomers may be of any type.

A pore typically contains (a) one construct comprising two monomers and (b) a sufficient number of monomers to form the pore. The construct may be any of those discussed above. The monomers may be any of those discussed above, including mutant monomers of the invention. Another typical pore comprises more than one construct of the invention, such as two, three or four constructs of the invention. Such pores further comprise a sufficient number of monomers to form the pore. The monomer may be any of those discussed above.

Mutations can be introduced into the construct as described above. The mutations may be alternating, i.e. the mutations are different for each monomer within a two monomer construct and the constructs are assembled as a homo-oligomer resulting in alternating modifications. Alternatively, the mutations may be neighbouring, i.e. identical mutations are introduced into two monomers in a construct and this is then oligomerised with different mutant monomers. One or more of the monomers of the invention in a construct-containing pore may be chemically-modified as discussed above.

### Producing pores of the invention

The invention also provides a method of producing a pore of the invention. The method comprises allowing at least one mutant monomer of the invention or at least one construct of the invention to oligomerise with a sufficient number of mutant aerolysin monomers of the invention, constructs of the invention or monomers derived from aerolysin to form a pore. If the method concerns making a homo-oligomeric pore of the invention, all of the monomers used in the method are mutant aerolysin monomers of the invention having the same amino acid sequence. If the method concerns making a hetero-oligomeric pore of the invention, at least one of the monomers is different from the others. Any of the embodiments discussed above with reference to the pores of the invention equally apply to the methods of producing the pores.

### Methods of sensing and/or characterising substrates

The invention provides a method of sensing and/or characterising a target substrate. The method comprises contacting the target substrate with a pore of the invention such that the target substrate moves through the pore. One or more characteristics of the target substrate are then measured as the substrate moves with respect to the pore using standard methods known in the art. One or more characteristics of the target substrate are preferably measured as the substrate moves through the pore. Steps (a) and (b) are preferably carried out with a potential applied across the pore. The applied potential may be a voltage potential, preferably the voltage is of at least 220 mV, preferably at least 230 mV, more preferably at least 240 mV, even more preferably at least 250 mV, most preferably at least 260 mV. In a preferred aspect, the voltage is up to 350 mV, such as 220 to 350 mV, 230 to 350 mV, 240 to 350 mV, 250 to 350 mV or 260 to 350 mV. Alternatively, the applied potential may be a chemical potential. An example of this is using a salt gradient across an amphiphilic layer.

The use of high voltage, such as described above, is made possible by the elimination of the noise cause by bases neighbouring the base that is sensed.

The method of the invention is for sensing and/or characterising a target substrate. The method is for sensing and/or characterising at least one substrate. The method may concern sensing and/or characterising two or more substrate.

The method may comprise sensing and/or characterising any number of substrate, such as 1, 2, 5, 10, 15, 20, 30, 40, 50, 100 or more substrate. The target substrate is preferably, but not limited to, a metal ion, an inorganic salt, a polymer, an amino acid, a peptide, a polypeptide, a protein, a nucleotide, an oligonucleotide, a polynucleotide, a dye, a bleach, a pharmaceutical, a diagnostic agent, a recreational drug, an explosive or an environmental pollutant. The method may concern sensing and/or characterising two or more substrate of the same type, such as two or more proteins, two or more nucleotides or two or more pharmaceuticals. Alternatively, the method may concern sensing and/or characterising two or more substrate of different types, such as one or more proteins, one or more nucleotides and one or more pharmaceuticals.

The target substrate can be secreted from cells. Alternatively, the target substrate can be a substrate that is present inside cells such that the substrate must be extracted from the cells before the invention can be carried out.

In certain embodiments, the substrate is preferably an amino acid, a peptide, a polypeptide and/or a protein. The amino acid, peptide, polypeptide or protein can be naturally-occurring or non-naturally occurring.

The polypeptide or protein can include within them synthetic or modified amino acids. A number of different types of modification to amino acids are known in the art. Suitable amino acids and modifications thereof are above. For the purposes of the invention, it is to be understood that the target substrate can be modified by any method available in the art.

The protein can be for instance an enzyme, an antibody, a hormone, a growth factor or a growth regulatory protein, such as a cytokine. The cytokine may be selected from interleukins, preferably IFN-1, IL-1, IL-2, IL-4, IL-5, IL-6, IL-10, IL-12 and IL-13, interferons, and other cytokines. The protein may be a bacterial protein, a fungal protein, a virus protein or a parasite-derived protein.

In certain embodiments, the target substrate is preferably a nucleotide, an oligonucleotide or a polynucleotide. A nucleotide typically contains a nucleobase, a sugar and at least one phosphate group. The nucleobase is typically heterocyclic. Nucleobases include, but are not limited to, purines and pyrimidines and more specifically adenine, guanine, thymine, uracil and cytosine. The sugar is typically a pentose sugar. Nucleotide sugars include, but are not limited to, ribose and deoxyribose. The nucleotide is typically a ribonucleotide or deoxyribonucleotide. The nucleotide typically contains a monophosphate, diphosphate or triphosphate. Phosphates may be attached on the 5' or 3' side of a nucleotide. Nucleotides include, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (HDP), uridine triphosphate (UTP), cytidine monophosphate (CMP), cytidine diphosphate (CDP), cytidine triphosphate (CTP), 5-methylcytidine monophosphate, 5-methylcytidine diphosphate, 5-methylcytidine triphosphate, 5-hydroxymethylcytidine monophosphate, 5-hydroxymethylcytidine diphosphate, 5-hydroxymethylcytidine triphosphate, cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP), 5-methyl-2'-deoxycytidine monophosphate, 5-methyl-2'-deoxycytidine diphosphate, 5-methyl-2'-deoxycytidine triphosphate, 5-hydroxymethyl-2'-deoxycytidine monophosphate, 5-hydroxymethyl-2'-deoxycytidine diphosphate and 5-hydroxymethyl-2' - deoxycytidine triphosphate.

The nucleotides are preferably selected from AMP, TMP, GMP, UMP, dAMP, dTMP, dGMP or dCMP. The nucleotides may be abasic (i.e. lacking a nucleobase). The nucleotides may contain additional modifications. In particular, suitable modified nucleotides include, but are not limited to, 2'-amino pyrimidines (such as 2'-amino cytidine and 2'-amino uridine), 2'-hyrdroxyl purines (such as , 2'-fluoro pyrimidines (such as 2'-fluorocytidine and 2'-fluoro uridine), hydroxyl pyrimidines (such as 5'-a-P-borano uridine), 2'-0-methyl nucleotides (such as 2'-0-methyl adenosine, 2'-0-methyl guanosine, 2'-0-methyl cytidine and 2'-0-methyl uridine), 4'-thio pyrimidines (such as 4'-thio uridine and 4'-thio cytidine) and nucleotides have modifications of the nucleobase (such as 5-pentynyl-2'-deoxy uridine, 5-(3-aminopropyl)-uridine and 1,6-diaminohexyl-N-5-carbamoylmethyl uridine).

Oligonucleotides are short nucleotide polymers which typically have 50 or fewer nucleotides, such 40 or fewer, 30 or fewer, 20 or fewer, 10 or fewer or 5 or fewer nucleotides. The oligonucleotides may comprise any of the nucleotides discussed above, including the basic and modified nucleotides.

The method of the invention is particularly suitable for sensing and/or characterising a target polynucleotide. A polynucleotide, such as a nucleic acid, is a macromolecule comprising two or more nucleotides. The polynucleotide or nucleic acid may comprise any combination of any nucleotides. The nucleotides can be naturally occurring or artificial. One or more nucleotides in the target polynucleotide can be oxidized or methylated. One or more nucleotides in the target polynucleotide may be damaged. One or more nucleotides in the target polynucleotide may be modified, for instance with a label or a tag. Suitable labels are described above.

The nucleotides in the polynucleotide may be attached to each other in any manner. The nucleotides are typically attached by their sugar and phosphate groups as in nucleic acids. The nucleotides may be connected via their nucleobases as in pyrimidine dimers. The polynucleotide may be single stranded or double stranded. At least a portion of the polynucleotide is may be double stranded. A single stranded polynucleotide may have one or more primers hybridised thereto and hence comprise one or more short regions of double stranded polynucleotide. The primers may be the same type of polynucleotide as the target polynucleotide or may be a different type of polynucleotide.

The polynucleotide can be a nucleic acid, such as deoxyribonucleic acid (DNA) or ribonucleic acid (RNA). The target polynucleotide can comprise one strand of RNA hybridized to one strand of DNA. The polynucleotide may be any synthetic nucleic acid known in the art, such as peptide nucleic acid (PNA), glycerol nucleic acid (GNA), threose nucleic acid (TNA), locked nucleic acid (LNA) or other synthetic polymers with nucleotide side chains. The whole or only part of the target polynucleotide may be characterised using this method.

The target polynucleotide can be of any length. For example, the polynucleotide can be at least 10, at least 50, at least 100, at least 150, at least 200, at least 250, at least 300, at least 400 or at least 500 nucleotide pairs in length. The polynucleotide can be 1000 or more nucleotide pairs, 5000 or more nucleotide pairs in length or 100000 or more nucleotide pairs in length.

The target substrate, such as a target polynucleotide, is present in any suitable sample. The invention is typically carried out on a sample that is known to contain or suspected to contain the target substrate, such as the target polynucleotide. Alternatively, the invention may be carried out on a sample to confirm the identity of one or more target substrates, such as one or more target polynucleotides, whose presence in the sample is known or expected. Preferably, the sample is in a liquid form.

The sample may be a biological sample. The invention may be carried out in vitro on a sample obtained from or extracted from any organism or microorganism. The organism or microorganism is typically archaean, prokaryotic or eukaryotic and typically belongs to one the five kingdoms: plantae, animalia, fungi, monera and protista. The invention may be carried out in vitro on a sample obtained from or extracted from any virus. The sample is preferably a fluid sample. The sample typically comprises a body fluid of the patient. The sample may be urine, lymph, saliva, mucus or amniotic fluid but is preferably blood, plasma or serum. Typically, the sample is human in origin, but alternatively it may be from another mammal animal such as from commercially farmed animals such as horses, cattle, sheep or pigs or may alternatively be pets such as cats or dogs.

The sample may be a non-biological sample. The non-biological sample is preferably a fluid sample. Examples of a non-biological sample include surgical fluids, water such as drinking water, sea water or river water, and reagents for laboratory tests.

The sample is typically processed prior to being assayed, for example by centrifugation or by passage through a membrane that filters out unwanted molecules or cells, such as red blood cells. The sample may be measured immediately upon being taken. The sample may also be typically stored prior to assay, preferably below -70° C.

The pore is typically present in a membrane. Any membrane may be used in accordance with the invention. Suitable membranes are well-known in the art. The membrane is preferably an amphiphilic layer. An amphiphilic layer is a layer formed from amphiphilic molecules, such as phospholipids, which have both at least one hydrophilic portion and at least one lipophilic or hydrophobic portion. The amphiphilic molecules may be synthetic or naturally occurring.

The amphiphilic layer may be a monolayer or a bilayer. The amphiphilic layer is typically a planar lipid bilayer or a supported bilayer. The amphiphilic layer is typically a lipid bilayer. Lipid bilayers are models of cell membranes and serve as excellent platforms for a range of experimental studies. For example, lipid bilayers can be used for in vitro investigation of membrane proteins by single-channel recording. Alternatively, lipid bilayers can be used as biosensors to detect the presence of a range of substances. The lipid bilayer may be any lipid bilayer. Suitable lipid bilayers include, but are not limited to, a planar lipid bilayer, a supported bilayer or a liposome.

In another preferred embodiment, the membrane is a solid state layer. A solid-state layer is not of biological origin. In other words, a solid state layer is not derived from or isolated from a biological environment such as an organism or cell, or a synthetically manufactured version of a biologically available structure. Solid state layers can be formed from both organic and inorganic materials including, but not limited to, microelectronic materials, insulating materials, organic and inorganic polymers such as polyamide, plastics such as Teflon^{®} or elastomers such as two-component addition-cure silicone rubber, and glasses, and provides therefore the advantage of a manufacturing process free of any biologically-derived material. The solid state layer may be formed from monatomic layers, such as graphene, or layers that are only a few atoms thick.

Accordingly, one aspect of the invention relates to a system comprising a membrane having at least one pore according to the invention spanning across the membrane thickness. Preferably, the system comprises at least two chambers comprising a liquid medium, said chambers being separated by said membrane. The system according to the invention is particularly adapted and configured to act as a sensor or otherwise as analytical means for sensing and/or characterising a target substrate such as a small molecule, a polynucleotide or a polypeptide.

The method is typically carried out using (i) an artificial amphiphilic layer comprising a pore, (ii) an isolated, naturally-occurring lipid bilayer comprising a pore, or (iii) a cell having a pore inserted therein. The method is typically carried out using an artificial amphiphilic layer, such as an artificial lipid bilayer. The layer may comprise other transmembrane and/or intramembrane proteins as well as other molecules in addition to the pore. The method of the invention is typically carried out in vitro.

The substrate, such as a target polynucleotide, may be coupled to the membrane. This may be done using any known method. If the membrane is an amphiphilic layer, such as a lipid bilayer (as discussed in detail above), the substrate, such as a target polynucleotide, is preferably coupled to the membrane via a polypeptide present in the membrane or a hydrophobic anchor present in the membrane. The hydrophobic anchor is preferably a lipid, fatty acid, sterol, carbon nanotube or amino acid. The substrate, such as a target polynucleotide, may be coupled directly to the membrane. The substrate, such as a target polynucleotide, is preferably coupled to the membrane via a linker. Preferred linkers include, but are not limited to, polymers, such as polynucleotides, polyethylene glycols (PEGs) and polypeptides.

The substrate such as a target polynucleotide, may be transiently coupled to an amphiphilic layer, such as a lipid bilayer using cholesterol or a fatty acyl chain. Any fatty acyl chain having a length of from 6 to 30 carbon atoms, such as hexadecanoic acid, may be used. In preferred embodiments, the substrate, such as a target polynucleotide, is coupled to an amphiphilic layer. Coupling of substrates, such as a target polynucleotide, to synthetic lipid bilayers has been carried out previously with various different tethering strategies.

Polynucleotides may be functionalized using a modified phosphoramidite in the synthesis reaction, which is easily compatible for the addition of reactive groups, such as thiol, cholesterol, lipid and biotin groups. These different attachment chemistries give a suite of attachment options for polynucleotides. Each different modification group tethers the polynucleotide in a slightly different way and coupling is not always permanent so giving different dwell times for the polynucleotide to the bilayer.

Coupling of polynucleotides can also be achieved by a number of other means provided that a reactive group can be added to the polynucleotide. The addition of reactive groups to either end of DNA has been reported previously.

A selection of chemical groups, such as biotin, thiols and fluorophores, can be added using terminal transferase to incorporate modified oligonucleotides to the 3' of ssDNA (Kumar, A., P. Tchen, et al. (1988). "Nonradioactive labelling of synthetic oligonucleotide probes with terminal deoxynucleotidyl transferase." Anal Biochem 169 (2): 376-82).

Alternatively, the reactive group could be considered to be the addition of a short piece of DNA complementary to one already coupled to the bilayer, so that attachment can be achieved via hybridisation. Ligation of short pieces of ssDNA have been reported using T4 RNA ligase I (Troutt, A. B., M. G. McHeyzer-Williams, et al. (1992). "Ligationanchored PCR: a simple amplification technique with single-sided specificity." Proc Natl Acad Sci U S A 89(20): 9823-5). Alternatively, either ssDNA or dsDNA could be ligated to native dsDNA and then the two strands separated by thermal or chemical denaturation. To native dsDNA, it is possible to add either a piece of ssDNA to one or both of the ends of the duplex, or dsDNA to one or both ends.

Then, when the duplex is melted, each single strand will have either a 5' or 3' modification if ssDNA was used for ligation or a modification at the 5' end, the 3' end or both if dsDNA was used for ligation. If the polynucleotide is a synthetic strand, the coupling chemistry can be incorporated during the chemical synthesis of the polynucleotide. For instance, the polynucleotide can be synthesized using a primer a reactive group attached to it.

A common technique for the amplification of sections of genomic DNA is using polymerase chain reaction (PCR). Here, using two synthetic oligonucleotide primers, a number of copies of the same section of DNA can be generated, where for each copy the 5' of each strand in the duplex will be a synthetic polynucleotide. By using an antisense primer that has a reactive group, such as a cholesterol, thiol, biotin or lipid, each copy of the target DNA amplified will contain a reactive group for coupling.

The pore used in the method of the invention is a pore of the invention (i.e. a pore comprising at least one mutant monomer of the invention or at least one construct of the invention). The pore may be chemically modified in any of the ways discussed above. The pore is preferably modified with a covalent adaptor that is capable of interacting with the target substrate as discussed above.

The method is preferably adapted for sensing and/or characterising a target polynucleotide and step (a) comprises contacting the target polynucleotide with the pore and possibly a polynucleotide binding protein so that the protein controls the movement of the target polynucleotide through the pore. The polynucleotide binding protein may be any protein that is capable of binding to the polynucleotide and controlling its movement through the pore.

The protein typically interacts with and modifies at least one property of the polynucleotide. The protein may modify the polynucleotide by cleaving it to form individual nucleotides or shorter chains of nucleotides, such as di- or trinucleotides. The moiety may modify the polynucleotide by orienting it or moving it to a specific position, i.e. controlling its movement.

The polynucleotide binding protein is preferably a polynucleotide handling enzyme. A polynucleotide handling enzyme is a polypeptide that is capable of interacting with and modifying at least one property of a polynucleotide. The enzyme may modify the polynucleotide by cleaving it to form individual nucleotides or shorter chains of nucleotides, such as di- or trinucleotides. The enzyme may modify the polynucleotide by orienting it or moving it to a specific position. The polynucleotide handling enzyme does not need to display enzymatic activity as long as it is capable of binding the target sequence and controlling its movement through the pore.

There are two main strategies for sequencing polynucleotides using nanopores, namely strand sequencing and processing enzyme sequencing. The method of the invention may concern either strand sequencing or processing enzyme sequencing.

In strand sequencing, the DNA is translocated through the nanopore either with or against an applied potential. DNA polymerase that act progressively or processively on double stranded DNA can be used on the cis side of the pore to feed the remaining single strand through under an applied potential or the trans side under a reverse potential. Likewise, a helicase that unwinds the double stranded DNA can also be used in a similar manner.

There are also possibilities for sequencing applications that require strand translocation against an applied potential, but the DNA must be first "caught" by the enzyme under a reverse or no potential. With the potential then switched back following binding the strand will pass cis to trans through the pore and be held in an extended conformation by the current flow. The single strand DNA exonucleases or single strand DNA dependent polymerases can act as molecular motors to pull the recently translocated single strand back through the pore in a controlled stepwise manner, trans to cis, against the applied potential.

In one embodiment, the method of sensing and/or characterising a target polynucleotide involves contacting the target sequence with a pore and a helicase enzyme. Any helicase may be used in the method. Helicases may work in two modes with respect to the pore. First, the method is preferably carried out using a helicase such that it controls movement of the target sequence through the pore with the field resulting from the applied voltage. In this mode the 5' end of the DNA is first captured in the pore, and the enzyme controls movement of the DNA into the pore such that the target sequence is passed through the pore with the field until it finally translocates through to the trans side of the bilayer.

Alternatively, the method is preferably carried out such that a helicase enzyme controls movement of the target sequence through the pore against the field resulting from the applied voltage. In this mode the 3' end of the DNA is first captured in the pore, and the enzyme controls movement of the DNA through the pore such that the target sequence is pulled out of the pore against the applied field until finally ejected back to the cis side of the bilayer.

In exonuclease sequencing, an exonuclease releases individual nucleotides from one end of the target polynucleotide and these individual nucleotides are identified as discussed below.

In another embodiment, the method of sensing and/or characterising a target polynucleotide involves contacting the target sequence with a pore and an exonuclease enzyme. Exonucleases are enzymes that typically latch onto one end of a polynucleotide and digest the sequence one nucleotide at a time from that end. The exonuclease can digest the polynucleotide in the 5' to 3' direction or 3' to 5' direction. The end of the polynucleotide to which the exonuclease binds is typically determined through the choice of enzyme used and/or using methods known in the art. Hydroxyl groups or cap structures at either end of the polynucleotide may typically be used to prevent or facilitate the binding of the exonuclease to a particular end of the polynucleotide.

The method involves contacting the polynucleotide with the exonuclease so that the nucleotides are digested from the end of the polynucleotide at a rate that allows characterization or identification of a proportion of nucleotides as discussed above.

The rate at which the exonuclease functions is typically slower than the optimal rate of a wild-type exonuclease. A suitable rate of activity of the exonuclease in the method of the invention involves digestion of from 0.5 to 1000 nucleotides per second, from 0.6 to 500 nucleotides per second, 0.7 to 200 nucleotides per second, from 0.8 to 100 nucleotides per second, from 0.9 to 50 nucleotides per second or 1 to 20 or 10 nucleotides per second. The rate is preferably 1, 10, 100, 500 or 1000 nucleotides per second.

The method of the invention involves in certain embodiments measuring one or more characteristics of the target substrate, such as a target polynucleotide. The method may involve measuring two, three, four or five or more characteristics of the target substrate, such as a target polynucleotide. For target polynucleotides, the one or more characteristics are preferably selected from (i) the length of the target polynucleotide, (ii) the identity of the target polynucleotide, (iii) the sequence of the target polynucleotide, (iv) the secondary structure of the target polynucleotide and (v) whether or not the target polynucleotide is modified. Any combination of (i) to (v) may be measured in accordance with the invention.

For (i), the length of the polynucleotide may be measured using the number of interactions between the target polynucleotide and the pore. For (ii), the identity of the polynucleotide may be measured in a number of ways. The identity of the polynucleotide may be measured in conjunction with measurement of the sequence of the target polynucleotide or without measurement of the sequence of the target polynucleotide. The former is straightforward; the polynucleotide is sequenced and thereby identified. The latter may be done in several ways. For instance, the presence of a particular motif in the polynucleotide may be measured (without measuring the remaining sequence of the polynucleotide). Alternatively, the measurement of a particular electrical and/or optical signal in the method may identify the target polynucleotide as coming from a particular source.

For (iii), the sequence of the polynucleotide can be determined as described previously. Suitable sequencing methods, particularly those using electrical measurements, are described in Stoddart D et al., Proc Natl Acad Sci, 12;106(19):7702-7, Lieberman KR et al, J Am Chem Soc. 2010;132(50):17961-72, and International Application WO 2000/28312.

For (iv), the secondary structure may be measured in a variety of ways. For instance, if the method involves an electrical measurement, the secondary structure may be measured using a change in dwell time or a change in current flowing through the pore. This allows regions of single-stranded and double-stranded polynucleotide to be distinguished.

For (v), the presence or absence of any modification may be measured. The method preferably comprises determining whether or not the target polynucleotide is modified by methylation, by oxidation, by damage, with one or more proteins or with one or more labels, tags or spacers. Specific modifications will result in specific interactions with the pore which can be measured using the methods described below. For instance, methylcyotsine may be distinguished from cytosine on the basis of the current flowing through the pore during its interaction with each nucleotide.

The invention also provides a method of estimating the sequence of a target polynucleotide. The invention further provides a method of sequencing a target polynucleotide. A variety of different types of measurements may be made. This includes without limitation: electrical measurements and optical measurements. Possible electrical measurements include: current measurements, impedance measurements, tunnelling measurements (Ivanov AP et al., Nano Lett. 201 1 Jan 12; 1I(I):279-85), and FET measurements (International Application WO 2005/124888). Optical measurements may be combined with electrical measurements (Soni GV et al., Rev Sci Instrum. 2010 Jan;81(I):014301). The measurement may be a transmembrane current measurement such as measurement of ionic current flowing through a pore of the invention.

Electrical measurements may be made using standard single channel recording equipment as describe in Stoddart D et al., Proc Natl Acad Sci, 12;106(19):7702-7, Lieberman KR et al, J Am Chem Soc. 2010; 132(50): 17961-72, and International Application WO 2000/28312. Alternatively, electrical measurements may be made using a multichannel system, for example as described in International Application WO 2009/077734 and International Application WO 2011/067559.

In a preferred embodiment, the method comprises:
(a) contacting the target polynucleotide with a pore of the invention and a polynucleotide binding protein such that the target polynucleotide moves through the pore and the binding protein controls the movement of the target polynucleotide through the pore; and
(b) measuring the current passing through the pore as the polynucleotide moves with respect to the pore wherein the current is indicative of one or more characteristics of the target polynucleotide and thereby characterising the target polynucleotide.

The methods may be carried out using any apparatus that is suitable for investigating a membrane/pore system in which a pore is inserted into a membrane. The method may be carried out using any apparatus that is suitable for transmembrane pore sensing. For example, the apparatus comprises a chamber comprising an aqueous solution and a barrier that separates the chamber into two sections. The barrier has an aperture in which the membrane containing the pore is formed. For instance, the methods may be carried out using the apparatus described in International Application WO 2008/102120.

The methods may involve measuring the current passing through the pore as the substrate, such as a target polynucleotide, moves with respect to the pore. Therefore, the apparatus may also comprise an electrical circuit capable of applying a potential and measuring an electrical signal across the membrane and pore. The methods may be carried out using a patch clamp or a voltage clamp. The methods preferably involve the use of a voltage clamp.

The methods of the invention may involve the measuring of a current passing through the pore as the substrate, such as a target polynucleotide, moves with respect to the pore. Suitable conditions for measuring ionic currents through transmembrane protein pores are known in the art. The method is typically carried out with a voltage applied across the membrane and pore. It is possible to increase discrimination between different nucleotides by a pore by using an increased applied potential.

The methods are typically carried out in the presence of any charge carriers, such as metal salts, for example alkali metal salt, halide salts, for example chloride salts, such as alkali metal chloride salt. Charge carriers may include ionic liquids or organic salts, for example tetramethyl ammonium chloride, trimethylphenyl ammonium chloride, phenyltrimethyl ammonium chloride, or 1-ethyl -3-methyl imidazolium chloride. In the exemplary apparatus discussed above, the salt is present in the aqueous solution in the chamber. Potassium chloride (KC1), lithium chloride (LiCI), sodium chloride (NaCl) or caesium chloride (CsCI) is typically used. The salt concentration may be at saturation. High salt concentrations provide a high signal to noise ratio and allow for currents indicative of the presence of a nucleotide to be identified against the background of normal current fluctuations.

The methods are typically carried out in the presence of a buffer. In the exemplary apparatus discussed above, the buffer is present in the aqueous solution in the chamber. Any suitable buffer may be used in the method of the invention. Typically, the buffer is HEPES. Another suitable buffer is Tris-HCl buffer. The methods are typically carried out at a pH of from 3.0 to 12.0, preferably about 7.5.

The methods may be carried out at from 0 ° C to 100 ° C, from 15 ° C to 95° C, from 16° C to 90° C, from 17° C to 85° C, from 18° C to 80° C, 19° C to 70° C, or from 20° C to 60° C. The methods are typically carried out at room temperature (around 25 ° C, such as between 20 and 30 ° C). The methods are optionally carried out at a temperature that supports enzyme function, such as about 37 ° C.

The method is typically carried out in the presence of free nucleotides or free nucleotide analogues and an enzyme cofactor that facilitate the action of the polynucleotide binding protein, such as a helicase or an exonuclease. The free nucleotides may be one or more of any of the individual nucleotides discussed above. The free nucleotides are preferably selected from AMP, TMP, GMP, CMP, UMP, dAMP, dTMP, dGMP or dCMP. The free nucleotides are preferably adenosine triphosphate (ATP). The enzyme cofactor is a factor that allows a processing enzyme to function. The enzyme cofactor is preferably a divalent metal cation. The divalent metal cation is preferably Mg, Mn, Ca or Co. The enzyme cofactor is most preferably Mg2+.

The target polynucleotide may be contacted with the pore and the polynucleotide binding protein in any order. In is preferred that, when the target polynucleotide is contacted with the protein and the pore, the target polynucleotide firstly forms a complex with the protein. When the voltage is applied across the pore, the target polynucleotide/protein complex then forms a complex with the pore and controls the movement of the polynucleotide through the pore.

### Methods of identifying an individual nucleotide

The present invention also provides a method of sensing and/or characterising an individual nucleotide. In other words, the target substrate is an individual nucleotide. The method comprises contacting the nucleotide with a pore of the invention such that the nucleotide interacts with the pore and measuring the current passing through the pore during the interaction and thereby characterizing the nucleotide. The invention therefore involves nanopore sensing of an individual nucleotide.

### Methods of forming sensors

The invention also provides a method of forming a sensor for sensing and/or characterising a target analyte such as a polynucleotide. In certain embodiments, the method comprises forming a complex between a pore of the invention and a polynucleotide binding protein, such as a helicase or an exonuclease. The complex may be formed by contacting the pore and the protein in the presence of the target analyte, such as a polynucleotide, and then applying a potential across the pore. The applied potential may be a chemical potential and/or a voltage potential as described above. Alternatively, the complex may be formed by covalently attaching the pore to the protein through methods known in the art.

The complex is a sensor for sensing and/or characterising the target analyte, such as a polynucleotide. The method preferably comprises forming a complex between a pore of the invention and a processing enzyme. Any of the embodiments discussed above equally apply to this method.

The invention also provides a sensor for sensing and/or characterising a target analyte such as a polynucleotide. The sensor comprises a complex between a pore of the invention and a polynucleotide binding protein. Any of the embodiments discussed above equally apply to the sensor of the invention.

### Apparatus

The invention also provides an apparatus for sensing and/or characterising, such as sequencing, target analyte such as a polynucleotide in a sample. The apparatus may comprise (a) a plurality of pores of the invention and (b) a plurality of polynucleotide binding proteins, such as helicases or exonucleases. The apparatus may be any conventional apparatus for analyte analysis, such as an array or a chip.

The apparatus preferably comprises: a sensor device that is capable of supporting the plurality of pores and being operable to perform polynucleotide characterising or sequencing using the pores and proteins; at least one reservoir for holding material for performing the characterising or sequencing; a fluidics system configured to controllably supply material from the at least one reservoir to the sensor device; and a plurality of containers for receiving respective samples, the fluidics system being configured to supply the samples selectively from the containers to the sensor device.

### EXAMPLES

### Example 1: Assessment of a sensing method using the aerolysin nanopore of SEQ ID NO:16.

### Methods

Nanopore experiments were conducted using the Orbit Mini equipment. Phospholipid bilayer was formed across 50- *µ* m orifice in a MECA 4 recording chip that contains a 2 x 2 array of circular microcavities, which separates each of chamber into two compartments, cis and trans (Figure 1). Each cavity contains an individual integrated Ag/AgCI-microelectrode, and is able to record four artificial lipid bilayers in parallel. The temperature of chamber was set to 25 degree if no specific description. Recombinant aerolysin mutants were expressed and purified as described in (Cao C. et al., Single-molecule sensing of peptides and nucleic acids by engineered aerolysin nanopores,Nat Commun. 2019; 10(1):4918). Purified protein was diluted to the concentration of 0.2 µg/ml and then incubated with trypsin-agarose for 2 h under 4° C. The solution was centrifuged to remove trypsin. The current traces were measured with EDR Recording and Analysis software at a sampling rate of 50~200 kHz and filtered at 2~100 kHz. The biotinylated ssDNA were incubated with the streptavidin firstly and then added in the cis chamber after applying a negative voltage from the cis side. When a voltage is applied across the lipid bilayer, transmembrane potential pulls the streptavidin-biotin-ssDNA complex thread through the pore from the cis compartment, reducing the open pore current to a specific state (close to 0 pA). The current value of this lower state depends on the type of nucleotides inside the pore, especially on the types of nucleotides around the constriction region. The ssDNA remains inside the pore until a reverse voltage is applied to eject it back to the cis chamber.

### Nucleotide current signature in engineered aerolysin monomer according to the invention

An aerolysin monomer was prepared having the amino acid sequence SEQ ID NO:16. The sequence was produced by introducing the following amino acids substitution into the amino acid sequence of SEQ ID NO:8. A pore was formed with seven monomers having the sequence SEQ ID NO:16.

The capability of the present pore for characterizing the current signature of homopolymers of adenine, cytosine, thymine, and guanine was assessed by means of single-channel recording experiments. The experimental system is shown in Figure 1a. It consists of a lipid membrane that separates a chamber into two compartments, cis and trans, with the tested aerolysin mutant pore connecting both compartments. Based on the molecular dynamic (MD) simulation results, the tested aerolysin nanopore has one single constriction site, at position R220. The diameter of this constriction site is ~1.0 nm and its length is ~0.5 nm (Figure 1a and Figure 2). Therefore, the tested aerolysin nanopore provides a significantly enhanced resolution for biomolecule recognition versus the wild-type aerolysin and also with respect to prior art aerolysin nanopore (data not shown). When a voltage was applied across the lipid membrane, transmembrane potential pulled the streptavidin-biotin-ssDNA complex thread through the pore from the cis chamber (Figure 1a), reducing the open pore current to a specific lower state (close to 0 pA). The current value of this lower state depends on the type of nucleotides occupying inside the pore, especially for the types of nucleotides around the constriction region. Figure 3 provides a raw current trace recording. The ssDNA remained inside the pore until a negative voltage was applied to eject it back to the cis chamber. Since more than 80% ssDNA prefer to enter aerolysin pore from 3'-terminal and the observation in MspA pore that there is a larger current distinction for 3' threaded homopolymers, the 3' threading was used in the present study. Therefore, the biotin-linker was attached to 5' terminals of all ssDNA used in this study. The biotinylated ssDNA could specifically bind to streptavidin and thus allow ssDNA to enter the pore from 3'terminals. The position order of each nucleotide in ssDNA were named from 5'-terminal. To ensure each ssDNA fully occupies the pore, the length for all ssDNA was set to 30 nucleobases.

Since the homopolymer guanine could not be detected due to G-tetrad formation, the residual current of homopolymers of adenine, cytosine, and thymine was measured firstly, i.e., dA30, dC30, and dT30. As shown in Figure 3, the residual current levels when ssDNA was immobilized inside the pore (state ②) was measured and then the mean residual current for each blockade event was calculated. For each homopolymer, the data from at least 5 different pores was used and each pore had at least 200 blockade events. Finally, the histograms of residual current were plotted and their distribution were fitted by the Gaussian function (Figure 4). The residual current of dA30 (left), dC30 (center), and dT30 (right) were well resolved without any overlap, locating at 4.5 pA, 5.2 pA and 8.6 pA, respectively. To cautiously check this observation, a mixture experiment of dA30 and dC30 was performed (Figure 5). Two histograms were obtained, one located at 4.5 pA and the other one at 8.6pA. Therefore, all results imply that three types of homopolymers (adenine, cytosine, thymine) generated specific current signatures in the tested aerolysin nanopore comprising the monomer of SEQ ID NO:16.

### Identification of the sensing region of the engineered aerolysin nanopore

Investigating the sensing region of the pore is crucial to study influence of neighbouring bases on the sequence reading. Although the MD results has shown that the monomer of SEQ ID NO:16 only has one constriction, an experiment was designed to probe its sensing region. Since different types of homo-oligonucleotides generate specific current signatures in the aerolysin monomer of SEQ ID NO: 16, and knowing that two main sensing regions of wt aerolysin are located at the pore entry and pore exit, an oligonucleotide was constructed in such a way that half of the bases inside the pore would be cysteine (those present in the region of the pore entry) and the other half would be adenine (those present near the pore exist). In a first step, it is necessary to determine how many bases are expected to enter the pore. In previous results (data not shown), 14 bases of free ssDNA fully blocked the aerolysin pore. In streptavidin-biotin-ssDNA system, such as used here, the number of bases inside the pore is a little bit higher than in a free ssDNA system, due to the influence of streptavidin-biotin complex. Considering how streptavidin might dock on the surface of aerolysin's pore entrance, it was assumed that 18 bases are able to occupy the entire lumen of the pore in the streptavidin-biotin-ssDNA system. Therefore, the oligonucleotide for the present assessment was built by replacing the 9 cysteine bases of the cysteine homopolymer, starting from the 5' terminal, by adenine bases. As shown in Figure 2a, nine adenines fully covered the first sensing region (R220) and the rest cytosine threading the second sensing region (K238). The oligonucleotide used in the present experiment therefore had the sequence 5'-dA9dC21. A parallel similar experiment was also performed with the sequence of 5'-dC9dA21. Upon application of a current, the results are expected to differ between a pore having one sensing region and a pore having two sensing regions. If there were two sensing regions, the measured current values of 5'-dA9dC21 and 5'-dC9dA21 should both be in the middle of the current values measured in the above-described experiment performed with dA30 and dC30. In the case of a pore with only one constriction at R220, the current value for 5'-dA9dC21 should be closer to dA30 whereas the current should be closer to dC30 if the constriction is located at 238 position. The current distributions (Figure 2b) demonstrated that current value of 5'-dA9dC21 is the same as dA30, while for 5'-dC9dA21 the current histogram is similar to the one of dC30. These results confirm the observation from MD simulation that the aerolysin nanopore based on the monomer of SEQ ID NO:16 only has one single constriction region and that this constriction site is at R220.

### Effect of neighbour bases on sequence reading

Having determined that the tested pore only had one single recognition site, a single cytosine, dC, was introduced into a homopolymer of adenine at the 2nd, 3rd, 4th, 5th, 6th, 7th, 8th, 9th, 12th, and 16th position, respectively, to study the influence of neighbor bases for the sequence reading, i.e the following ssDNA oligonucleotides were prepared: 5'-dAdCdA28, 5'-dA2dCdA27, 5'-dA3dCdA26, 5'-dA4dCdA25, 5'-dA5dCdA24, 5'-dA6dCdA23, 5'-dA7dCdA22, 5'-dA8dCdA21, 5'-dA11dCdA18 and 5'-dA15dCdA14. Meanwhile, a single dA and a single dT were introduced at the same positions into a homopolymer of cytosine (thus producing the ssDNA sequences 5'-dCdAdC28, 5'-dC2dAdC27, 5'-dC3dAdC26, 5'-dC4dAdC25, 5'-dC5dAdC24, 5'-dC6dAdC23, and 5'-dC7dTdC22). The oligonucleotides were introduced into the pore and sensed. As shown in Figure 6, the residual current of each ssDNA was plotted against the position of substituted nucleotides. The data was collected at the condition of 1.0 M KCI solution buffered with 10 mM Tris and 1.0 mM EDTA, titrated to pH=7.4 under the voltage of 140 mV. For homopolymers of adenine, the data suggested that four nucleobases influence the reading, while only two bases influenced the reading in the case of the cytosine homopolymer. Additionally, the current was only altered when the substituted amino acid was in positions from 5 to 9. The rest of the sequence does not impact the current value, again suggesting that the tested engineered pore only has one reading point.

### Example 2: Increasing sensing accuracy by higher voltages

To study the influence of neighbouring bases on current reading in the engineered aerolysin nanopores assessed in Example 1, oligonucleotides were produced having a single dC substitution at each of positions 3 to 7 of a homopolymer of adenine. The current values of each of these oligonucleotides was mapped at different voltages. As shown in Figure 7, under 140 mV, the current values are similar when dC is in the 6th and 7th position. In contrast, with an increased voltage of 180 mV was used, the current value was closer to the homopolymer of cytosine when dC was in the 6th position, but there were still four bases influencing the current reading. Surprisingly, when the voltages was increased above 220 mV, only one position determined the value of current, i.e. the 5th position. More importantly, under 260 mV condition, the current value of 5'-dA4dCdA25 was exactly the same as that of dC30, suggesting that the base in the 5^{th} position could be sensed with a single base accuracy with no influence of the neighbouring bases in position 4 and 6. For comparison, the current differences between the highest value of ssDNA with one dC substitution and dC30 were shown by the box on the right of Figure 7. The height of the box represents the value of current differences.

These results demonstrate a revolutionary improvement of nanopore reading accuracy for DNA sequencing which is the bottleneck in nanopore sequencing field. For comparison purpose, in Mycobacterium smegmatis porin A (MspA) nanopore, for example, there are four bases influence, resulting in 256 identifiable current levels. In contrast, in the present engineered aerolysin system with a nanopore based on the aerolysin monomer of SEQ ID NO:16 and a current of 260 mV, only one base determines the reading, resulting in only 4 distinct current levels, each level corresponding to one base.

### SEQUENCE LISTING

**SEQ ID NO: 1** >sp|P09167|AERA_AERHY Aerolysin OS=Aeromonas hydrophila GN=aerA 
AEPVYPDQLRLFSLGQGVCGDKYRPVNREEAQSVKSNIVGMMGQWQISG
LANGWVIMGPGYNGEIKPGTASNTWCYPTNPVTGEIPTLSALDIPDGDEV
DVQWRLVHDSANFIKPTSYLAHYLGYAWVGGNHSQYVGEDMDVTRDGD
GWVIRGNNDGGCDGYRCGDKTAIKVSNFAYNLDPDSFKHGDVTQSDRQ
LVKTVVGWAVNDSDTPQSGYDVTLRYDTATNWSKTNTYGLSEKVTTKN
KFKWPLVGETELSIEIAANQSWASQNGGSTTTSLSQSVRPTVPARSKIPVK
IELYKADISYPYEFKADVSYDLTLSGFLRWGGNAWYTHPDNRPNWNHTF
VIGPYKDKASSIRYQWDKRYIPGEVKWWDWNWTIQQNGLSTMQNNLAR
VLRPVRAGITGDFSAESQFAGNIEIGAPVPLAADSKVRRARSVDGAGQGLR
LEIPLDAQELSGLGFNNVSLSVTPAANQ
**SEQ ID NO: 2** >sp|Q06306|AER5_AERHY Aerolysin-5 OS=Aeromonas hydrophila GN=ahh5 
AEPVYPDQLRLFSLGQEVCGDKYRPITREEAQSVKSNIVNMMGQWQISGL
ANGWVIMGPVYNGEIKPGSASNTWCYPVNPVTGEIPTLSALDIPDGDEVD
VQWRLVHDSANFIKPTSYLAHYLGYAWVGGNHSQYVGEDMDVTRDGDG
WVIRGNNDGGCEGYRCGEKTAIKVSNFAYNLDPDSFKHGDVTQSDRQLV
KTVVGWAINDSYTPQSAYDVTLRYDTATNWSKTNTYGLSEKVTTKNKFK
WPLVGETELSIEIAANQSWASQNGGSTTTSLSQSVRPTVPARSKIPVKIEL
YKADISYPYEFKADVSYDLTLSGFLRWGGNAWYTHPDNRPNWNHTFVIG
PYKDKASSIRYQWDKRYIPGEVKWWDWNWTIQQNGLSTMQNNLARVLR
PVRAGITGDFSAESQFAGNIEIGAPVPLAADGKAPRALSARRGEQGLRLAIP
LECRKSSPGLASATSA
**SEQ ID NO: 3** >sp|Q06304|AERA_AERSO Aerolysin OS=Aeromonas sobria GN=asa1 
AEPVYPDQVKWAGLGTGVCASGYRPLTRDEAMSIKGNLVSRMGQWQIT
GLADRWVIMGPGYNGEIKQGTAGETWCYPNSPVSGEIPTLSDWNIPAGD
EVDVQWRLVHDNDYFIKPVSYLAHYLGYAWVGGNHSPYVGEDMDVTRV
GDGWLIKGNNDGGCSGYRCGEKSSIKVSNFSYTLEPDSFSHGQVTESGK
QLVKTITANATNYTDLPQQVVVTLKYDKATNWSKTDTYSLSEKVTTKNK
FQWPLVGETELAIEIAASQSWASQKGGSTTETVSVEARPTVPPHSSLPVRV
ALYKSNISYPYEFKAEVNYDLTMKGFLRWGGNAWYTHPDNRPTWEHTL
LLGPFRGQGEQHPLPVDKRYIPGEVKWWDWNWTISEYGLSTMQNNLGR
VLRPIRSAVTGDFYAESQFAGDIEIGQPQTRSAKAAQLRSASAEEVALTSV
DLDSEALANEGFGNVSLTIVPVQ
**SEQ ID NO: 4** >sp|Q06305|AER3_AERHY Aerolysin-3 OS=Aeromonas hydrophila GN=ahh3 
AEPVYPDQLRLFSLGQEVCGDKYRPVNREEAQSVKSNIVGMMGQWQISG
LANGWVIMGPGYNGEIKPGSASSTWCYPTNPATGEIPTLSALDIPDGDEV
DVQWRLVHDSANFIKPTSYLAHYLGYAWVGGNHSQYVGEDMDVTRDGD
GWVIRGNNDGGCDGYRCGDKTSIKVSNFAYNLDPDSFKHGDVTQSDRQ
LVKTVVGWAINDSDTPQSGYDVTLRYDTATNWSKTNTYGLSEKVTTKNK
FKWPLVGETELSIEIAANQSWASQNGGSPTTSLSQSVRPTVPAHSKIPVKI
ELYKADISYPYEFKADVSYDLTLSGFLRWGGNAWYTHPDNRPNWNHTF
VIGPYKDKASSIRYQWDKRYIPGEVKWWDWNWTIQQNGLPTMQNNLAK
VLRPVRAGITGDFSAESQFAGNIEIGAPVPVAAASHSSRARNLSAGQGLRL
EIPLDAQELSGLGFNNVSLSVTPAANQ
**SEQ ID NO: 5** >sp|P09166|AERA_AEREN Aerolysin OS=Aeromonas enteropelogenes GN=aerA 
NAAEPIYPDQLRLFSLGEDVCGTDYRPINREEAQSVRNNIVAMMGQWQIS
GLANNWVILGPGYNGEIKPGKASTTWCYPTRPATAEIPVLPAFNIPDGDA
VDVQWRMVHDSANFIKPVSYLAHYLGYAWVGGDHSQFVGDDMDVIQEG
DDWVLRGNDGGKCDGYRCNEKSSIRVSNFAYTLDPGSFSHGDVTQSERT
 
LVHTVVGWATNISDTPQSGYDVTLNYTTMSNWSKTNTYGLSEKVSTKNK
FKWPLVGETEVSIEIAANQSWASQNGGAVTTALSQSVRPVVPARSRVPVKI
ELYKANISYPYEFKADMSYDLTFNGFLRWGGNAWHTHPEDRPTLSHTFA
IGPFKDKASSIRYQWDKRYLPGEMKWWDWNWAIQQNGLATMQDSLAR
VLRPVRASITGDFRAESQFAGNIEIGTPVPLGSDSKVRRTRSVDGANTGLK
LDIPLDAQELAELGFENVTLSVTPARN
**SEQ ID NO: 6** >sp|Q08676|AERA_AERSA Aerolysin OS=Aeromonas salmonicida GN=ash3 
WHEPVYPDQVKWAGLGTGVCASGYRPLTRDEAMSIKGNLVSRMGQWQI
TGLADRWVIMGPGYNGEIKQGTAGETWCYPNSPVSGEIPTLSDWNIPAG
DEVDVQWRLVHDNDYFIKPVSYLAHYLGYAWVGGNHSPYVGEDMDVTR
VGDGWLIKGNNDGGCSGYRCGEKSSIKVSNFSYTLEPDSFSHGQVTESG
KQLVKTITANATNYTDLPQQVVVTLKYDKATNWSKTDTYSLSEKVTTKN
KFQWPLVGETELAIEIAASQSWASQKGGSTTETVSVEARPTVPPHSSLPV
RVALYKSNISYPYEFKAEVNYDLTMKGFLRWGGNAWYTHPDNRPTWEH
TFRLGPFRGQGEQHPLPVDKRYIPGEVKWWDWNWTISEYGLSTMQNNL
GRVLRPIRSAVTGDFYAESQFAGDIEIGQPQTRSAKAAQLRSASAEEVALT
SVDLDSEALANEGFGNVSLTIVPVQ
**SEQ ID NO: 7** >sp|Q06303|AER4_AERHY Aerolysin-4 OS=Aeromonas hydrophila GN=ahh4 
AEPVYPDQLRLFSLGQEVCGDKYRPVNREEAQSIKSNIVGMMGQWQISG
LANGWVIMGPGYNGEIKPGSASSTWCYPTNPATGEIPTLSALDIPDGDEV
DVQWRLVHDSANFIKPTSYLAHYLGYAWVGGNHSQYVGEDMDVTRDGD
GWVIRGNNDGGCDGYRCGDKTSIKVSNFAYNLDPDSFKHGDVTQSDRQ
LVKTVVGWAINDSDTPQSGYDVTLRYDTATNWSKTNTYGLSEKVTTKNK
FKWPLVGETELSIEIAANQSWASQNGGSTTTSLSQSVRPTVPAHSKIPVKI
ELYKADISYPYEFKADVSYDLTLSGFLRWGGNAWYTHPDNRPNWNHTF
VIGPYKDKASSIRYQWDKRYIPGEVKWWDWNWTIQQNGLPTMQNNLAK
 
VLRPVRAGITGDFSAESQFAGNIEIGAPVPVAAASHSSRARNLSAGQGLRL
EIPLDAQELSGLGFNNVSLSVTPAANQ
**SEQ ID NO: 8** >sp|P09167|AERA_AERHY Aerolysin OS=Aeromonas hydrophila GN=aerA without C-terminal propeptide 
AEPVYPDQLRLFSLGQGVCGDKYRPVNREEAQSVKSNIVGMMGQWQISG
LANGWVIMGPGYNGEIKPGTASNTWCYPTNPVTGEIPTLSALDIPDGDEV
DVQWRLVHDSANFIKPTSYLAHYLGYAWVGGNHSQYVGEDMDVTRDGD
GWVIRGNNDGGCDGYRCGDKTAIKVSNFAYNLDPDSFKHGDVTQSDRQ
LVKTVVGWAVNDSDTPQSGYDVTLRYDTATNWSKTNTYGLSEKVTTKN
KFKWPLVGETELSIEIAANQSWASQNGGSTTTSLSQSVRPTVPARSKIPVK
IELYKADISYPYEFKADVSYDLTLSGFLRWGGNAWYTHPDNRPNWNHTF
VIGPYKDKASSIRYQWDKRYIPGEVKWWDWNWTIQQNGLSTMQNNLAR
VLRPVRAGITGDFSAESQFAGNIEIGAPVPL
**SEQ ID NO: 9** >cds for P09167|AERA_AERHY Aerolysin OS=Aeromonas hydrophila GN=aerA (including C-terminal peptide) 
gcagagcccg tctatccaga ccagcttcgc ttgttttcat tgggccaagg ggtctgtggc
gacaagtatc gccccgtcaa tcgagaagaa gcccaaagcg ttaaaagcaa tattgtcggc
atgatggggc aatggcaaat aagcgggctg gccaacggct gggtcattat ggggccgggt
tataacggtg aaataaaacc agggacagcg tccaatacct ggtgttatcc gaccaatcct
gttaccggtg aaataccgac actgtctgcc ctggatattc cagatggtga cgaagtcgat
gtgcagtggc gactggtaca tgacagtgcg aatttcatca aaccaaccag ctatctggcc
cattacctcg gttatgcctg ggtgggcggc aatcacagcc aatatgtcgg cgaagacatg
gatgtgaccc gtgatggcga cggctgggtg atccgtggca acaatgacgg cggctgtgac
ggctatcgct gtggtgacaa gacggccatc aaggtcagca acttcgccta taacctggat
cccgacagct tcaagcatgg cgatgtcacc cagtccgacc gccagctggt caagactgtg
gtgggctggg cggtcaacga cagcgacacc ccccaatccg gctatgacgt caccctgcgc
tacgacacag ccaccaactg gtccaagacc aacacctatg gcctgagcga gaaggtgacc
accaagaaca agttcaagtg gccactggtg ggggaaaccc aactctccat cgagattcgt
 
gccaatcagt cctgggcgtc ccagaacggg ggctcgacca ccacctccct gtctcagtcc
gtgcgaccga ctgtgccggc ccgctccaag atcccggtga agatagagct ctacaaggcc
gacatctcct atccctatga gttcaaggcc gatgtcagct atgacctgac cctgagcggc
ttcctgcgct ggggcggcaa cgcctggtat acccacccgg acaaccgtcc gaactggaac
cacaccttcg tcataggtcc gtacaaggac aaggcgagca gcattcgcta ccagtgggac
aagcgttaca tcccgggtga agtgaagtgg tgggactgga actggaccat acagcagaac
ggtctgtcta ccatgcagaa caacctggcc agagtgctgc gcccggtgcg ggcggggatc
accggtgatt tcagtgccga gagccagttt gccggcaaca tagagatcgg tgctcccgtg
ccgctcgcgg ctgacagcaa ggtgcgtcgt gctcgcagtg tggacggcgc tggtcaaggc
ctgaggctgg agatcccgct cgatcgcgaa gagctctccg ggcttggctt caacaagtca
gcctcagcgt ga
**SEQ ID NO: 10** >sp|Q06306|AER5_AERHY Aerolysin-5 OS=Aeromonas hydrophila GN=ahh5 without C-terminal propeptide 
AEPVYPDQLRLFSLGQEVCGDKYRPITREEAQSVKSNIVNMMGQW
QISGLANGWVIMGPVYNGEIKPGSASNTWCYPVNPVTGEIPTLSALDIPD
GDEVDVQWRLVHDSANFIKPTSYLAHYLGYAWVGGNHSQYVGEDMDVT
RDGDGWVIRGNNDGGCEGYRCGEKTAIKVSNFAYNLDPDSFKHGDVTQ
SDRQLVKTVVGWAINDSYTPQSAYDVTLRYDTATNWSKTNTYGLSEKVT
TKNKFKWPLVGETELSIEIAANQSWASQNGGSTTTSLSQSVRPTVPARSKI
PVKIELYKADISYPYEFKADVSYDLTLSGFLRWGGNAWYTHPDNRPNWN
HTFVIGPYKDKASSIRYQWDKRYIPGEVKWWDWNWTIQQNGLSTMQNN
LARVLRPVRAGITGDFSAESQFAGNIEIGAPVPL
**SEQ ID NO: 11** >sp|Q06304|AERA_AERSO Aerolysin OS=Aeromonas sobria GN=asa1 without C-terminal propeptide 
AEPVYPDQVKWAGLGTGVCASGYRPLTRDEAMSIKGNLVSRMGQWQIT
GLADRWVIMGPGYNGEIKQGTAGETWCYPNSPVSGEIPTLSDWNIPAGD
EVDVQWRLVHDNDYFIKPVSYLAHYLGYAWVGGNHSPYVGEDMDVTRV
GDGWLIKGNNDGGCSGYRCGEKSSIKVSNFSYTLEPDSFSHGQVTESGK
 
QLVKTITANATNYTDLPQQVVVTLKYDKATNWSKTDTYSLSEKVTTKNK
FQWPLVGETELAIEIAASQSWASQKGGSTTETVSVEARPTVPPHSSLPVRV
ALYKSNISYPYEFKAEVNYDLTMKGFLRWGGNAWYTHPDNRPTWEHTL
LLGPFRGQGEQHPLPVDKRYIPGEVKWWDWNWTISEYGLSTMQNNLGR
VLRPIRSAVTGDFYAESQFAGDIEIGQPQ
**SEQ ID NO: 12** >sp|Q06305|AER3_AERHY Aerolysin-3 OS=Aeromonas hydrophila GN=ahh3 without C-terminal propeptide 
AEPVYPDQLRLFSLGQEVCGDKYRPVNREEAQSVKSNIVGMMGQWQISG
LANGWVIMGPGYNGEIKPGSASSTWCYPTNPATGEIPTLSALDIPDGDEV
DVQWRLVHDSANFIKPTSYLAHYLGYAWVGGNHSQYVGEDMDVTRDGD
GWVIRGNNDGGCDGYRCGDKTSIKVSNFAYNLDPDSFKHGDVTQSDRQ
LVKTVVGWAINDSDTPQSGYDVTLRYDTATNWSKTNTYGLSEKVTTKNK
FKWPLVGETELSIEIAANQSWASQNGGSPTTSLSQSVRPTVPAHSKIPVKI
ELYKADISYPYEFKADVSYDLTLSGFLRWGGNAWYTHPDNRPNWNHTF
VIGPYKDKASSIRYQWDKRYIPGEVKWWDWNWTIQQNGLPTMQNNLAK
VLRPVRAGITGDFSAESQFAGNIEIGAPVPV
**SEQ ID NO: 13** >sp|P09166|AERA_AEREN Aerolysin OS=Aeromonas enteropelogenes GN=aerA without C-terminal propeptide 
NAAEPIYPDQLRLFSLGEDVCGTDYRPINREEAQSVRNNIVAMMGQWQIS
GLANNWVILGPGYNGEIKPGKASTTWCYPTRPATAEIPVLPAFNIPDGDA
VDVQWRMVHDSANFIKPVSYLAHYLGYAWVGGDHSQFVGDDMDVIQEG
DDWVLRGNDGGKCDGYRCNEKSSIRVSNFAYTLDPGSFSHGDVTQSERT
LVHTVVGWATNISDTPQSGYDVTLNYTTMSNWSKTNTYGLSEKVSTKNK
FKWPLVGETEVSIEIAANQSWASQNGGAVTTALSQSVRPVVPARSRVPVKI
ELYKANISYPYEFKADMSYDLTFNGFLRWGGNAWHTHPEDRPTLSHTFA
IGPFKDKASSIRYQWDKRYLPGEMKWWDWNWAIQQNGLATMQDSLAR
VLRPVRASITGDFRAESQFAGNIEIGTPVPL
**SEQ ID NO: 14** >sp|Q08676|AERA_AERSA Aerolysin OS=Aeromonas salmonicida GN=ash3 without C-terminal propeptide 
WHEPVYPDQVKWAGLGTGVCASGYRPLTRDEAMSIKGNLVSRMGQWQI
TGLADRWVIMGPGYNGEIKQGTAGETWCYPNSPVSGEIPTLSDWNIPAG
DEVDVQWRLVHDNDYFIKPVSYLAHYLGYAWVGGNHSPYVGEDMDVTR
VGDGWLIKGNNDGGCSGYRCGEKSSIKVSNFSYTLEPDSFSHGQVTESG
KQLVKTITANATNYTDLPQQVVVTLKYDKATNWSKTDTYSLSEKVTTKN
KFQWPLVGETELAIEIAASQSWASQKGGSTTETVSVEARPTVPPHSSLPV
RVALYKSNISYPYEFKAEVNYDLTMKGFLRWGGNAWYTHPDNRPTWEH
TFRLGPFRGQGEQHPLPVDKRYIPGEVKWWDWNWTISEYGLSTMQNNL
GRVLRPIRSAVTGDFYAESQFAGDIEIGQPQ
**SEQ ID NO: 15** >sp|Q06303|AER4_AERHY Aerolysin-4 OS=Aeromonas hydrophila GN=ahh4 without C-terminal propeptide 
AEPVYPDQLRLFSLGQEVCGDKYRPVNREEAQSIKSNIVGMMGQWQISG
LANGWVIMGPGYNGEIKPGSASSTWCYPTNPATGEIPTLSALDIPDGDEV
DVQWRLVHDSANFIKPTSYLAHYLGYAWVGGNHSQYVGEDMDVTRDGD
GWVIRGNNDGGCDGYRCGDKTSIKVSNFAYNLDPDSFKHGDVTQSDRQ
LVKTVVGWAINDSDTPQSGYDVTLRYDTATNWSKTNTYGLSEKVTTKNK
FKWPLVGETELSIEIAANQSWASQNGGSTTTSLSQSVRPTVPAHSKIPVKI
ELYKADISYPYEFKADVSYDLTLSGFLRWGGNAWYTHPDNRPNWNHTF
VIGPYKDKASSIRYQWDKRYIPGEVKWWDWNWTIQQNGLPTMQNNLAK
VLRPVRAGITGDFSAESQFAGNIEIGAPVPV
**SEQ ID NO: 16** Aerolysin mutant monomer K238A 
AEPVYPDQLRLFSLGQGVCGDKYRPVNREEAQSVKSNIVGMMGQWQISG
LANGWVIMGPGYNGEIKPGTASNTWCYPTNPVTGEIPTLSALDIPDGDEV
DVQWRLVHDSANFIKPTSYLAHYLGYAWVGGNHSQYVGEDMDVTRDGD
GWVIRGNNDGGCDGYRCGDKTAIKVSNFAYNLDPDSFKHGDVTQSDRQ
LVKTVVGWAVNDSDTPQSGYDVTLRYDTATNWSKTNTYGLSEAVTTKN
 
KFKWPLVGETELSIEIAANQSWASQNGGSTTTSLSQSVRPTVPARSKIPVK
IELYKADISYPYEFKADVSYDLTLSGFLRWGGNAWYTHPDNRPNWNHTF
VIGPYKDKASSIRYQWDKRYIPGEVKWWDWNWTIQQNGLSTMQNNLAR
VLRPVRAGITGDFSAESQFAGNIEIGAPVPLAADSKVRRARSVDGAGQGLR
LEIPLDAQELSGLGFNNVSLSVTPAANQ
**SEQ ID NO: 17** Aerolysin mutant monomer K238A&K242A 
AEPVYPDQLRLFSLGQGVCGDKYRPVNREEAQSVKSNIVGMMGQWQISG
LANGWVIMGPGYNGEIKPGTASNTWCYPTNPVTGEIPTLSALDIPDGDEV
DVQWRLVHDSANFIKPTSYLAHYLGYAWVGGNHSQYVGEDMDVTRDGD
GWVIRGNNDGGCDGYRCGDKTAIKVSNFAYNLDPDSFKHGDVTQSDRQ
LVKTVVGWAVNDSDTPQSGYDVTLRYDTATNWSKTNTYGLSEAVTTAN
KFKWPLVGETELSIEIAANQSWASQNGGSTTTSLSQSVRPTVPARSKIPVK
IELYKADISYPYEFKADVSYDLTLSGFLRWGGNAWYTHPDNRPNWNHTF
VIGPYKDKASSIRYQWDKRYIPGEVKWWDWNWTIQQNGLSTMQNNLAR
VLRPVRAGITGDFSAESQFAGNIEIGAPVPLAADSKVRRARSVDGAGQGLR
LEIPLDAQELSGLGFNNVSLSVTPAANQ
**SEQ ID NO: 18** nucleotide sequence encoding for the polypeptide of SEQ ID NO:16 
gcagagcccg tctatccaga ccagcttcgc ttgttttcat tgggccaagg ggtctgtggc
gacaagtatc gccccgtcaa tcgagaagaa gcccaaagcg ttaaaagcaa tattgtcggc
atgatggggc aatggcaaat aagcgggctg gccaacggct gggtcattat ggggccgggt
tataacggtg aaataaaacc agggacagcg tccaatacct ggtgttatcc gaccaatcct
gttaccggtg aaataccgac actgtctgcc ctggatattc cagatggtga cgaagtcgat
gtgcagtggc gactggtaca tgacagtgcg aatttcatca aaccaaccag ctatctggcc
cattacctcg gttatgcctg ggtgggcggc aatcacagcc aatatgtcgg cgaagacatg
gatgtgaccc gtgatggcga cggctgggtg atccgtggca acaatgacgg cggctgtgac
ggctatcgct gtggtgacaa gacggccatc aaggtcagca acttcgccta taacctggat
cccgacagct tcaagcatgg cgatgtcacc cagtccgacc gccagctggt caagactgtg
 
gtgggctggg cggtcaacga cagcgacacc ccccaatccg gctatgacgt caccctgcgc
tacgacacag ccaccaactg gtccaagacc aacacctatg gcctgagcga ggccgtgacc
accaagaaca agttcaagtg gccactggtg ggggaaaccc aactctccat cgagattcgt
gccaatcagt cctgggcgtc ccagaacggg ggctcgacca ccacctccct gtctcagtcc
gtgcgaccga ctgtgccggc ccgctccaag atcccggtga agatagagct ctacaaggcc
gacatctcct atccctatga gttcaaggcc gatgtcagct atgacctgac cctgagcggc
ttcctgcgct ggggcggcaa cgcctggtat acccacccgg acaaccgtcc gaactggaac
cacaccttcg tcataggtcc gtacaaggac aaggcgagca gcattcgcta ccagtgggac
aagcgttaca tcccgggtga agtgaagtgg tgggactgga actggaccat acagcagaac
ggtctgtcta ccatgcagaa caacctggcc agagtgctgc gcccggtgcg ggcggggatc
accggtgatt tcagtgccga gagccagttt gccggcaaca tagagatcgg tgctcccgtg
ccgctcgcgg ctgacagcaa ggtgcgtcgt gctcgcagtg tggacggcgc tggtcaaggc
ctgaggctgg agatcccgct cgatcgcgaa gagctctccg ggcttggctt caacaagtca
gcctcagcgt ga
**SEQ ID NO: 19** nucleotide sequence encoding for the polypeptide of SEQ ID NO:17

gcagagcccg tctatccaga ccagcttcgc ttgttttcat tgggccaagg ggtctgtggc
gacaagtatc gccccgtcaa tcgagaagaa gcccaaagcg ttaaaagcaa tattgtcggc
atgatggggc aatggcaaat aagcgggctg gccaacggct gggtcattat ggggccgggt
tataacggtg aaataaaacc agggacagcg tccaatacct ggtgttatcc gaccaatcct
gttaccggtg aaataccgac actgtctgcc ctggatattc cagatggtga cgaagtcgat
gtgcagtggc gactggtaca tgacagtgcg aatttcatca aaccaaccag ctatctggcc
cattacctcg gttatgcctg ggtgggcggc aatcacagcc aatatgtcgg cgaagacatg
gatgtgaccc gtgatggcga cggctgggtg atccgtggca acaatgacgg cggctgtgac
ggctatcgct gtggtgacaa gacggccatc aaggtcagca acttcgccta taacctggat
cccgacagct tcaagcatgg cgatgtcacc cagtccgacc gccagctggt caagactgtg
gtgggctggg cggtcaacga cagcgacacc ccccaatccg gctatgacgt caccctgcgc
tacgacacag ccaccaactg gtccaagacc aacacctatg gcctgagcga ggccgtgacc
accgccaaca agttcaagtg gccactggtg ggggaaaccc aactctccat cgagattcgt
gccaatcagt cctgggcgtc ccagaacggg ggctcgacca ccacctccct gtctcagtcc

gtgcgaccga ctgtgccggc ccgctccaag atcccggtga agatagagct ctacaaggcc
gacatctcct atccctatga gttcaaggcc gatgtcagct atgacctgac cctgagcggc
ttcctgcgct ggggcggcaa cgcctggtat acccacccgg acaaccgtcc gaactggaac
cacaccttcg tcataggtcc gtacaaggac aaggcgagca gcattcgcta ccagtgggac
aagcgttaca tcccgggtga agtgaagtgg tgggactgga actggaccat acagcagaac
ggtctgtcta ccatgcagaa caacctggcc agagtgctgc gcccggtgcg ggcggggatc
accggtgatt tcagtgccga gagccagttt gccggcaaca tagagatcgg tgctcccgtg
ccgctcgcgg ctgacagcaa ggtgcgtcgt gctcgcagtg tggacggcgc tggtcaaggc
ctgaggctgg agatcccgct cgatcgcgaa gagctctccg ggcttggctt caacaagtca
gcctcagcgt ga

## Claims

1. A mutant polypeptide selected from
a. a polypeptide comprising a first amino acid substitution selected from the group consisting of K238A, K238S, K238G and K238D of the amino acid sequence of any one of SEQ ID NO:1, 2, 4, 6, 7, 8, 10, 12, 14 or 15 or a variant or fragment thereof; and
b. a polypeptide comprising a first amino acid substitution selected from the group consisting of V238A, V238S, V238G and V238D of the amino acid sequence of any one of SEQ ID NO:3 or 11 or a variant or fragment thereof;

2. A mutant polypeptide according to claim 1 or a polypeptide according to SEQ ID NO:5 or 13, comprising at least one of
- a second amino acid substitution selected from the group consisting of K242W and K242A, preferably K242A when the sequence is selected from any one of SEQ ID NO:1, 2, 4, 6, 7, 8, 10, 12, 14 and 15 of variants or fragments thereof or a second amino acid substitution selected from the group consisting of N242W and N242A, preferably N242A, when the sequence is selected from any one of SEQ ID NO:3 or 11 or a variant or fragment thereof or a second amino acid substitution selected from the group consisting of S242W and S242A, preferably S242A, when the sequence is selected from any one of SEQ ID NO:5 or 13 or a variant or fragment thereof.
- a third amino acid substitution selected from the group consisting of R282A, R282S, R282G, R282D, R282W, preferably selected from the group consisting of R282A and R282S, most preferably R282S when the sequence is selected from any one of SEQ ID NO:1,2, 4, 6 to 8, 10, 12, 14 and 15 or a variant or fragment thereof or a third amino acid substitution selected from P282A, P282S, P282G, P282D, P282W, preferably selected from the group consisting of P282A and P282S, most preferably P282S when the sequence is selected from SEQ ID NO:3 and 11 or a variant or fragment thereof;
- a fourth amino acid substitution selected from the group consisting of D216A, D216S, D216G, D216D and D216W, preferably selected from the group consisting of D216A and D216S, most preferably D216S when the sequence is selected from any one of SEQ ID NO:1, 2, 4, 7, 8, 10, 12 and 15 or a variant or fragment thereof or a fourth amino acid substitution selected from selected from the group consisting of V216A, V216S, V216G, V216D and V216W, preferably selected from the group consisting of V216A and V216S, most preferably V216S when the sequence is selected from SEQ ID NO: 3, 6, 11 or 14 or a variant or fragment thereof or a fourth amino acid substitution selected from selected from the group consisting of G216A and G216S, most preferably V216S when the sequence is selected from SEQ ID NO: 5 and 13;
- a fifth amino acid substitution selected from the group consisting of D222A, D222S, D222G and D222D, preferably selected from the group consisting of D222A and D222S, most preferably D222S when the sequence is selected from any one of SEQ ID NO:1,2, 4, 6 to 8, 10, 12, 14 and 15 or a variant or fragment thereof or a fifth amino acid substitution selected the group consisting of K222A, K222S, K222G and K222D, preferably selected from the group consisting of K222A and K222S, most preferably K222S when the sequence is selected from SEQ ID NO:3 or 11 or a variant or fragment thereof or a fifth amino acid substitution selected from the group consisting of N222A, N222S, N222G and N222D, preferably selected from the group consisting of N222A and N222S, most preferably N222S when the sequence is selected from any one of SEQ ID NO: 5 and 13;
- a sixth amino acid substitution selected from the group consisting of E258A, E258S, E258G, E258D and E258W, preferably selected from the group consisting of E258A and E258S, most preferably E258S when the sequence is selected from any one of SEQ ID NO:1, 2, 4, 6 to 8, 10, 12, 14 and 15 or a variant or fragment thereof or a sixth amino acid substitution selected from the group consisting of I258A, I258S, I258G, I258D and I258W, preferably selected from the group consisting of I258A and I258S, most preferably I258S when the sequence is selected from any one of SEQ ID NO: 3 and 11 or a variant or fragment thereof;
- a seventh amino acid substitution selected from the group consisting of E254A, E254S, E254G, E254D and E254W, preferably selected from the group consisting of E254A and E254S, most preferably E254S when the sequence is selected from any one of SEQ ID NO:1,2, 4, 5, 6 to 8, 10, 12, 14 and 15 or a variant or fragment thereof or a seventh amino acid substitution selected from the group consisting of L254A, L254S, L254G, L254D and L254W, preferably selected from the group consisting of L254A and L254S, most preferably L254S when the sequence is selected from any one of SEQ ID NO: 3 and 11 or a variant or fragment thereof;
- an eighth amino acid substitution selected from the group consisting of K244A, K244S, K244G, K244D and K244W, preferably selected from the group consisting of K244A and K244S, most preferably K244S when the sequence is selected from any one of SEQ ID NO:1, 2, 4, 5, 6 to 8, 10, 12, 14 and 15 or a variant or fragment thereof or an eighth amino acid substitution selected from the group consisting of F244A, F244S, K244G, F244D and F244W, preferably selected from the group consisting of F244A and F244S, most preferably F244S when the sequence is selected from any one of SEQ ID NO: 3 and 11 or a variant or fragment thereof;
- a ninth amino acid substitution selected from the group consisting of E252A, E252S, E252G, E252D and E252W, preferably selected from the group consisting of E252A and E252S, most preferably E252S when the sequence is selected from any one of SEQ ID NO:1, 2, 4, 6 to 8, 10, 12, 14 and 15 or a variant or fragment thereof or a ninth amino acid substitution selected from the group consisting of T252A, T252S, T252G, T252D and T252W, preferably selected from the group consisting of T252A and T252S, most preferably T252S when the sequence is selected from any one of SEQ ID NO: 3 and 11 or a variant or fragment thereof or a ninth amino acid substitution selected from the group consisting of V252A, V252S, V252G, V252D and V252W, preferably selected from the group consisting of V252A and V252S, most preferably V252S when the sequence is selected from any one of SEQ ID NO: 5 and 13 or a variant or fragment thereof; and
- a tenth amino acid substitution selected from the group consisting of K246S, K246A K246G, K246D and K246W, preferably selected from the group consisting of K246A and K246S, most preferably K246S when the sequence is selected from any one of SEQ ID NO:1, 2, 4, 5, 7, 8, 10, 12, 13, and 15 or a variant or fragment thereof or a tenth amino acid substitution selected from the group consisting of W246S, W246A W246G, W246D and W246W, preferably selected from the group consisting of W246A and W246S, most preferably W246S when the sequence is selected from any one of SEQ ID NO: 3 and 11 or a variant or fragment thereof or a tenth amino acid substitution selected from the group consisting of Q246S, Q246A Q246G, Q246D and Q246W, preferably selected from the group consisting of Q246A and Q246S, most preferably Q246S when the sequence is selected from any one of SEQ ID NO: 6 and 14 or a variant or fragment thereof.

3. A polypeptide according to claim 2 selected from the group consisting of:
- a polypeptide comprising at least a first and a second amino acid substitution, preferably comprising exactly a first and a second amino acid substitution;
- a polypeptide comprising at least a first, a second and a third amino acid substitution, preferably comprising exactly a first a second and a third amino acid substitution;
- a polypeptide comprising comprising at least a first, a second, a third and a fourth amino acid substitution, preferably comprising exactly a first, a second, a third and a fourth amino acid substitution;
- a polypeptide comprising at least a first, a second, a third, a fourth and a fifth amino acid substitutions, preferably comprising exactly a first, a second, a third, a fourth and a fifth amino acid substitution;
- a polypeptide comprising at least a first, a second, a third, a fourth, a fifth and a sixth amino acid substitution, preferably comprising exactly at least a first, a second, a third, a fourth, a fifth and a sixth amino acid substitution;
- a polypeptide comprising at least a first, a second, a third, a fourth, a fifth, a sixth and a seventh amino acid substitution, preferably comprising exactly a first, a second, a third, a fourth, a fifth, a sixth and a seventh amino acid substitution;
- a polypeptide comprising at least a first, a second, a third, a fourth, a fifth, a sixth, a seventh and an eighth amino acid substitution, preferably comprising exactly a first, a second, a third, a fourth, a fifth, a sixth, a seventh and an eighth amino acid substitution;
- a polypeptide comprising at least a first, a second, a third, a fourth, a fifth, a sixth, a seventh, an eighth and a ninth amino acid substitution, preferably comprising exactly a first, a second, a third, a fourth, a fifth, a sixth, a seventh, an eighth and a ninth amino acid substitution; and
- a polypeptide comprising a first, a second, a third, a fourth, a fifth, a sixth, a seventh, an eighth, a ninth and a tenth amino acid substitution, preferably comprising exactly a first, a second, a third, a fourth, a fifth, a sixth, a seventh, an eighth, a ninth and a tenth amino acid substitution.

4. An aerolysin monomer consisting of a polypeptide according to any one of claims 1 to 3, wherein the amino acid sequence is any one of SEQ ID NO:8 or 10 to 15 or a variant or fragment thereof, preferably any one of SEQ ID NO:8 and 10 to 15, more preferably SEQ ID NO:8.

5. An aerolysin monomer according to claim 4, having a constriction site at position 220 of the amino acid sequence **characterized by** a diameter of at most 1.2 nm, more preferably at most 1.1 nm, most preferably at most 1.0 nm and/or by a height of at most 0.7 nm, preferably at most 0.6 nm, most preferably at most 0.5 nm.

6. A mutant aerolysin pore comprising at least one aerolysin monomer according to any one claims 4 to 6.

7. A construct comprising:
- two or more covalently attached monomers derived from said mutant aerolysin monomer according to any one of claims 4 to 6;
- a homo-oligomeric pore derived from said mutant aerolysin monomer according to any one of claims 4 to 6 comprising identical mutant monomers; or
- a hetero-oligomeric pore derived from said mutant aerolysin monomer according to any one of claims 4 to 6, wherein at least one of the monomers differs from the others.

8. A method of sensing and/or characterising a target substrate comprising:
(a) contacting the target substrate with a mutant aerolysin pore according to claim 7 so to allow the movement of the target substrate through said pore and a portion of the target substrate interacts with said pore; and
(b) measuring a current passing through said pore, thereby sensing and/or characterising the target substrate.

9. The method of claim 6, wherein steps (a) and (b) are carried out with a voltage applied across the pore.

10. The method according to claim 10, wherein the voltage applied across the pore is of is of at least 220 mV, preferably at least 230 mV, more preferably at least 240 mV, even more preferably at least 250 mV, most preferably at least 260 mV.

11. The method according to any one of claims 9 to 11, wherein
- the target substrate is a nucleic acid, and wherein said contacting is controlled by handling enzyme so that the movement of the nucleic acid through the pore and a proportion of the nucleotides in the target sequence interacts with the pore; or
- wherein the target substrate is a metal ion, an inorganic salt, a polymer, an amino acid, a peptide, a polypeptide, a protein, a nucleotide, an oligonucleotide, a polynucleotide, a dye, a bleach, a pharmaceutical, a diagnostic agent, a recreational drug, an explosive or an environmental pollutant.

12. An apparatus for sensing a target substrate in a sample, comprising the mutant aerolysin pore of claim 7.

13. The apparatus of claim 13, wherein the target substrate is a nucleic acid sequence, further comprising a nucleic acid handling enzymes.

14. A system comprising a membrane having at least one mutant aerolysin pore according to claim 7 spanning across the membrane thickness.

15. A nucleic acid encoding a polypeptide according to any one of claims 1 to 3.
